# EUROPEAN PATENT APPLICATION

(11) **EP 3 466 460 A1**
(43) Date of publication of application: **10.04.2019**
(21) Application number: 18198326.3
(22) Date of filing: 02.10.2018
(51) Int. Cl.: A61M 1/28, A61M 1/16

(54) **PERITONEAL DIALYSATE TEMPERATURE REGULATION SYSTEM**

(30) Priority: 03.10.2017 US 201715723550
(71) Applicant: Medtronic, Inc., Minneapolis, Minnesota 55432 (US)
(72) Inventor: GERBER, Martin T., Maple Grove, MN Minnesota 55369 (US); HOBOT, Christopher M., Rogers, MN Minnesota 55374 (US); Lura, David B., Maple Grove,, MN Minnesota 55311 (US)
(74) Representative: Maschio, Antonio

(57) **Abstract**

The invention relates to devices, systems, and methods for temperature regulation in generating peritoneal dialysate and using a peritoneal dialysis (PD) system. The system can use a heater and temperature sensors to regulate the temperature of fluid within the PD system.

## Description

### CROSS REFERENCE TO RELATED APPLICATIONS

This application is a continuation in part of U.S. Patent Application No. 15/478,569 filed April 4, 2017, which claims benefit of and priority to U.S. Provisional Application No. 62/318,173 filed April 4, 2016, and the disclosures of each of the above-identified applications are hereby incorporated by reference in their entirety.

### FIELD OF THE INVENTION

The invention relates to devices, systems, and methods for temperature regulation in generating peritoneal dialysate and using a peritoneal dialysis (PD) system. The system can use a heater and temperature sensors to regulate the temperature of fluid within the PD system.

### BACKGROUND

Peritoneal Dialysis (PD), including Automated Peritoneal Dialysis (APD) and Continuous Ambulatory Peritoneal Dialysis (CAPD) can be performed at a clinic or in a home-setting either by a patient alone or with a care-giver. PD differs from Hemodialysis (HD) in that blood is not removed from the body and passed through a dialyzer, but rather a catheter is placed in the peritoneal cavity and dialysate introduced directly into the peritoneal cavity. Blood is cleaned inside the patient using the patient's own peritoneum as a type of dialysis membrane. However, because fluid is directly introduced into a human body, the fluid used for peritoneal dialysate is generally required to be free of biological and chemical contaminants. The peritoneal dialysate should also contain specified concentrations of solutes and cations for biocompatibility and for performing membrane exchange.

Known systems and methods cannot both generate peritoneal dialysate and perform dialysis. The known systems and methods cannot generate and infuse peritoneal dialysate to a patient. Additionally, known systems and methods cannot adjust the temperature of a fluid use to make the peritoneal dialysate inside the system to change the solubility of solutes and cations for generating the peritoneal dialysate. The known systems and methods cannot clean, disinfect or flush the system. The known systems and methods also cannot heat a fluid or a disinfectant solution inside the system. In general, the known systems and methods cannot provide proper temperature regulation during dialysate generation, cleaning, disinfection, and therapy.

As such, there is a need for systems and methods that can control the temperature of a fluid for generating a peritoneal dialysate, and safely delivering and removing the dialysate from a patient during therapy. The systems and methods should control the temperature of a fluid during peritoneal dialysate generation for patient safety and comfort. There is also a need for systems and methods that can generate the required temperatures during dialysate generation and therapy, as well as during cleaning and disinfection in systems that can use a reusable fluid loop. There is also a need for control systems, related sensors, and flow paths for controlling temperatures during each of the described modalities.

### SUMMARY OF THE INVENTION

The first aspect of the invention relates to a temperature regulation system for use in peritoneal dialysis. In any embodiment, the temperature regulation system can comprise a first fluid line fluidly connected to a dialysate generation system; the first fluid line including a heater and a first temperature sensor positioned downstream of the heater; the first fluid line fluidly connectable to an integrated cycler through a second fluid line having a second temperature sensor; a control system in communication with the first temperature sensor, the second temperature sensor, and the heater; the control system controlling the heater based on a temperature measured by the first temperature sensor and the second temperature sensor.

In any embodiment, the first fluid line is fluidly connected to at least one osmotic agent source downstream of the heater.

In any embodiment, the system can comprise a sterilization module positioned between the first fluid line and the osmotic agent source.

In any embodiment, the sterilization module can comprise one or more ultrafilters.

In any embodiment, the dialysate generation system can be fluidly connectable to a disinfectant source.

In any embodiment, the dialysate generation system can be fluidly connectable to a waste line; the waste line fluidly connectable to the integrated cycler.

In any embodiment, the system can comprise a system pump; the system pump recirculating fluid from the disinfectant source, through the dialysate generation system, the temperature regulation system, and the second fluid line.

In any embodiment, the control system can control the heater based on a temperature measured by the first temperature sensor and/or the second temperature sensor.

The features disclosed as being part of the first aspect of the invention can be in the first aspect of the invention, either alone or in combination, or follow a preferred arrangement of one or more of the described elements.

The second aspect of the invention is directed to a method comprising the steps of generating a peritoneal dialysate with a peritoneal dialysate generation system; pumping the peritoneal dialysate through a temperature regulation system; the temperature regulation system comprising at least a heater and a first temperature sensor; pumping the peritoneal dialysate into an integrated cycler through at least one fluid line; the at least one fluid line having a second temperature sensor; measuring a temperature of the peritoneal dialysate in the at least one fluid line; and heating the peritoneal dialysate with the heater to a desired temperature as measured by the first temperature sensor and/or the second temperature sensor.

In any embodiment, the desired temperature as measured by the first temperature sensor can be between around 25°C and around 40 °C.

In any embodiment o, the desired temperature as measured by the second temperature sensor can be between around 25°C and around 40 °C.

In any embodiment, the method can comprise the step of pumping the peritoneal dialysate from the integrated cycler.

In any embodiment, the method can comprise the step of generating an alarm if a temperature of the peritoneal dialysate as measured by the first temperature sensor and/or the second temperature sensor is outside of a predetermined range.

In any embodiment of the second aspect of the invention, the method can comprise stopping the step of pumping the peritoneal dialysate from the integrated cycler if a temperature of the peritoneal dialysate as measured by the first temperature sensor and/or the second temperature sensor is outside of a predetermined range.

In any embodiment, the predetermined range as measured by the first temperature sensor can be between around 25°C and around 40 °C.

In any embodiment, the predetermined range as measured by the second temperature sensor can be between around 25°C and around 40 °C.

The features disclosed as being part of the second aspect of the invention can be in the second aspect of the invention, either alone or in combination, or follow a preferred arrangement of one or more of the described elements.

The third aspect of the invention is drawn to a method of disinfecting a peritoneal dialysis system, comprising the steps of: (a) fluidly connecting a disinfectant source to a first fluid line of a peritoneal dialysate generation system; wherein the peritoneal dialysate generation system is fluidly connected to a temperature regulation system and a second fluid line; the second fluid line fluidly connectable to an integrated cycler; (b) fluidly connecting the first fluid line to the second fluid line; (c) recirculating a disinfectant through the peritoneal dialysis system; (d) measuring a temperature of the disinfectant in the peritoneal dialysis system; and (e) heating the disinfectant to a desired temperature as measured by a first temperature sensor positioned in the first fluid line downstream of a heater and/or a second temperature sensor positioned in the second fluid line.

In any embodiment, the desired temperature as measured by the first temperature sensor can be equal to or greater than around 80 °C.

In any embodiment, the desired temperature as measured by the second temperature sensor can be equal to or greater than around 80 °C.

In any embodiment, the disinfectant can be a citric acid solution.

The features disclosed as being part of the third aspect of the invention can be in the third aspect of the invention, either alone or in combination, or follow a preferred arrangement of one or more of the described elements.

### BRIEF DESCRIPTION OF THE DRAWINGS

FIG. 1 shows a peritoneal dialysate generation flow path with an integrated cycler.
FIG. 2 shows a system for adding concentrates to a peritoneal dialysate generation flow path.
FIG. 3 shows an overview of a system for generating and using peritoneal dialysate with a single concentrate source.
FIG. 4 shows an overview of a system for generating and using peritoneal dialysate with multiple concentrate sources.
FIG. 5 shows an alternative peritoneal dialysate generation flow path with an integrated cycler.
FIG. 6 shows a peritoneal dialysate generation flow path with multiple dispensing options.
FIG.'s 7A-D show a peritoneal dialysate generation cabinet with a water reservoir and waste reservoir.
FIG. 8 shows a peritoneal dialysate generation cabinet connected to a faucet and drain.
FIG. 9 shows a peritoneal dialysate generation and delivery system.
FIG. 10 shows an exemplary system for temperature regulation and control.

### DETAILED DESCRIPTION OF THE INVENTION

Unless defined otherwise, all technical and scientific terms used have the same meaning as commonly understood by one of ordinary skill in the art.

The articles "a" and "an" are used to refer to one or to over one (*i.e*., to at least one) of the grammatical object of the article. For example, "an element" means one element or over one element.

The term "around," when used in the context of parameter values or ranges, means approximately, or within a certain margin from, the described values or ranges and should be given the broadest interpretation as understood by those of skill in the art.

A "citric acid solution" is a solution of citric acid, C₆H₈O₇, dissolved in water.

The term "communication" refers to an electronic or wireless link between two components.

The term "comprising" includes, but is not limited to, whatever follows the word "comprising." Use of the term indicates the listed elements are required or mandatory but that other elements are optional and may be present.

The term "consisting of' includes and is limited to whatever follows the phrase "consisting of." The phrase indicates the limited elements are required or mandatory and that no other elements may be present.

The term "consisting essentially of' includes whatever follows the term "consisting essentially of' and additional elements, structures, acts or features that do not affect the basic operation of the apparatus, structure or method described.

The terms "control," "controlling," or "controls" refers to the ability of one component to direct the actions of a second component.

A "control system" can be a combination of components acting together to maintain a system to a desired set of performance specifications. The control system can use processors, memory and computer components configured to interoperate to maintain the desired performance specifications. The control system can also include fluid or gas control components, and solute control components as known within the art to maintain the performance specifications.

The term "desired temperature" refers to an intended temperature of a fluid, a gas, or a combination thereof.

The term "dialysate" describes a fluid into or out of which solutes from a fluid to be dialyzed diffuse through a membrane. For example, for peritoneal dialysis, solutes can be diffused through a peritoneal membrane of a patient. Dialysate can differ depending on the type of dialysis to be carried out. For example, dialysate for peritoneal dialysis may include different solutes or different concentrations of solutes than dialysate for hemodialysis.

The term "dialysate generation system" refers to a set of components capable of creating dialysate for infusion into a patient.

The term "disinfectant solution" refers to any solution capable of destroying or removing bacterial contaminants from a reusable sorbent module.

The term "disinfectant source" refers to any source of a disinfectant solution. In certain embodiments, the disinfectant source can contain citric acid, bleach, or other disinfectants capable of removing biological contaminants.

The terms "disinfecting" or to "disinfect" refers to destroying biological contaminants in a fluid, gas, component, or system.

The term "downstream" refers to a position of a first component in a flow path relative to a second component wherein fluid, gas, or combination thereof, will pass by the second component prior to the first component during normal operation. The first component can be said to be "downstream" of the second component, while the second component is "upstream" of the first component.

The term "fluid" can be any substance without a fixed shape that yields easily to external pressure such as a gas or a liquid. Specifically, the fluid can be water containing any solutes at any concentration. The fluid can also be dialysate of any type including fresh, partially used, or spent.

The terms "fluid connection," "fluidly connectable," or "fluidly connected" refer to the ability to pass fluid or gas from one point to another point. The two points can be within or between any one or more of compartments, modules, systems, and components, all of any type.

A "fluid line" can refer to a tubing or conduit through which a fluid, gas, or fluid containing gas can pass. The fluid line can also contain air during different modes of operation such as cleaning or purging of a line.

The term "generating an alarm" or to "generate an alarm" can refer to generating or signaling to a user a state or condition of a system.

The terms to "generate peritoneal dialysate," "generating peritoneal dialysate" or "peritoneal dialysate generation" refers to creating a peritoneal dialysate solution from constituent parts.

A "heater" is a component capable of raising the temperature of a substance, container, fluid, or gas.

The terms "heating" or to "heat" refer to raising the temperature of a substance, fluid, gas, or container.

An "integrated cycler" is a component for movement of fluid into and out of the peritoneal cavity of a patient, wherein the integrated cycler forms a part of an overall system. For example, the integrated cycler can be contained in a housing with other components used for peritoneal dialysis and be in fluid and electrical connection with desired components.

The term "measuring" or "to measure" can refer to determining any parameter or variable. The parameter or variable can relate to any state or value of a system, component, fluid, gas, or mixtures of one or more gases or fluids.

An "osmotic agent source" refers to a source of osmotic agents in solid and/or solution form. The osmotic agent source can interface with at least one other module found in systems for dialysis. The osmotic agent source can contain at least one fluid pathway and include components such as conduits, valves, filters or fluid connection ports, any of which are fluidly connectable to each other or to a fluid flow path. The osmotic agent source can either be formed as a stand-alone enclosure or a compartment integrally formed with an apparatus for dialysis for containing an osmotic agent source. If the osmotic agent(s) is in solid form, a system as described in the present invention can deliver a fluid, such as a highly purified or sterile water, to dilute the solid osmotic agent. Optional mechanical agitation or other means such as stirring can be used to help dissolve the solid osmotic agent.

The term "peritoneal cavity" refers to the space between the parietal peritoneum and visceral peritoneum of a patient.

"Peritoneal dialysate" is a dialysis solution to be used in peritoneal dialysis having specified parameters for purity and sterility. Peritoneal dialysate is not the same as dialysate used in hemodialysis although peritoneal dialysate may be used in hemodialysis.

"Peritoneal dialysis" is a therapy wherein a dialysate is infused into the peritoneal cavity, which serves as a natural dialyzer. In general, waste components diffuse from a patient's bloodstream across a peritoneal membrane into the dialysis solution via a concentration gradient. In general, excess fluid in the form of plasma water flows from a patient's bloodstream across a peritoneal membrane into the dialysis solution via an osmotic gradient. Once the infused peritoneal dialysis solution has captured sufficient amounts of the waste components the fluid is removed. This cycle can be repeated for several cycles each day or as needed.

The term "predetermined range" is a range of possible values for a parameter to be set as.

The term "pump" refers to any device that causes the movement of fluids or gases by applying suction or pressure.

The terms "pumping fluid" or to "pump fluid" refer to moving a fluid or gas through a flow path with a pump.

"Purified water" can be defined as water produced by distillation, deionization, reverse osmosis, or other suitable processes and that meets the definition of "purified water" in the United States Pharmacopeia, 23d Revision, January 1, 1995, and the FDA at 21 CFR Section § 165.110(a)(2)(iv). Other criteria for purified water can be determined by those of skill in the art, particularly as relating to purified water suitable for peritoneal dialysis.

The terms "recirculating" or to "recirculate" refer to pumping fluid, gas, or a combination thereof, through a closed loop.

A "sterilization module" is a component or set of components to sterilize a fluid, gas, or combination thereof by removing or destroying chemical or biological contaminants.

The terms "stopping" or to "stop" refer to ceasing an action.

The term "system pump" refers to a pump for pumping fluids, gases, or combinations thereof through a peritoneal dialysis system.

The term "temperature regulation system" refers to a set of components that can measure and control a temperature of a fluid, gas, or a combination thereof.

A "temperature sensor" is a sensor capable of determining the temperature of a fluid, gas, or combination thereof.

An "ultrafilter" is a semi permeable membrane through which a fluid can pass with removal of one or more solutes or particles from the fluid.

The term "upstream" refers to a position of a first component in a flow path relative to a second component wherein fluid, gas, or combinations thereof, will pass by the first component prior to the second component during normal operation. The first component can be said to be "upstream" of the second component, while the second component is "downstream" of the first component.

The term "waste line" refers to a fluid line through which waste fluids or gases, or spent dialysate, can be pumped.

### Peritoneal Dialysis Temperature Regulation System

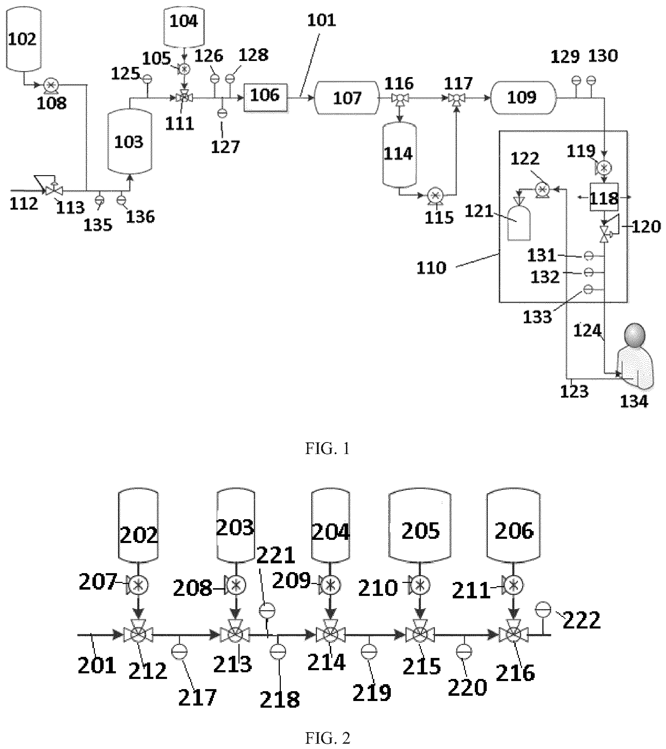

The first and second aspects of the invention relate to systems and methods for generating and using peritoneal dialysate in peritoneal dialysis. A system for generating peritoneal dialysate and delivering peritoneal dialysis therapy to a patient **134** can be configured as illustrated in FIG. 1. The system includes a peritoneal dialysate generation flow path **101**. Fluid from a water source, such as water tank **102**, can be pumped into the peritoneal dialysate generation flow path **101**. Additionally, or as an alternative to a water tank **102**, the system can use a direct connection **112** to a water source. System pump **108** can control the movement of fluid through the peritoneal dialysate generation flow path **101**. If a direct connection **112** to a water source is used, a pressure regulator **113** ensures the incoming water pressure is within a predetermined range. The system pumps the fluid from water source through a water purification module **103** to remove chemical contaminants in the fluid in preparation for creating dialysate.

The water source can be a source of potable water including a purified water source. Purified water can refer to any source of water treated to remove at least some biological or chemical contaminants. The water tank **102** can alternatively be a non-purified water source, such as tap water, wherein the water from the water tank **102** can be purified by the system as described. A non-purified water source can provide water that has undergone no additional purification, water that has undergone some level of purification, but does not meet the definition of "purified water" provided, such as bottled water or filtered water. The peritoneal dialysate generation flow path **101** can also have a direct connection **112** to a purified or non-purified water source, shown as direct connection **112**. The water source can be any source of water, whether from a tap, faucet, or a separate container or reservoir.

The water purification module **103** can be a sorbent cartridge. The sorbent cartridge can include aluminum oxide for removal of fluoride and heavy metals. The sorbent cartridge can have a first layer of aluminum oxide, a second layer of activated carbon and a third layer of an ion exchange resin. The sorbent cartridge can be sized depending on the needs of the user, with a larger sized sorbent cartridge allowing for more exchanges before the sorbent cartridge must be replaced. The sorbent cartridge can also include activated carbon. The activated carbon operates to adsorb non-ionic molecules, organic molecules, and chlorine from the water, along with some endotoxins or bacterial contaminants. In certain embodiments, the sorbent cartridge can include activated carbon, activated alumina, and potentially other components that work primarily by physical and chemical adsorption, combined with one or more ion exchange materials. The ion exchange materials can be any known material in the art, but preferably the ion exchange materials will release hydrogen and hydroxyl ions in exchange for other cations and anions in solution, resulting in water formation by the exchange process.

The sorbent cartridge can additionally include a microbial filter and/or a particulate filter. A microbial filter can further reduce the amount of bacterial contaminants present in the fluid from the water tank **102** or direct connection **112**. Optionally, an ultrafilter can be included to remove endotoxins from the fluid. A particulate filter can remove particulate matter from the fluid. The water tank **102** can be any size usable with the system, including between 12 and 25 L. A water tank **102** of 20 L can generally generate the necessary peritoneal dialysate for multiple cycles. In certain embodiments, the water purification module **103** can include an optional UV light source for further purification and sterilization of the water prior to adding osmotic agents or ion concentrates.

Alternatively, the water purification module **103** can be any component capable of removing contaminants from the water in the water source, including any one or more of a sorbent cartridge, reverse osmosis module, nanofilter, combination of cation and anion exchange materials, activated carbon, activated alumina, silica, or silica based columns.

After the fluid passes through the water purification module **103**, the fluid is pumped to a concentrate source **104**, where necessary components for carrying out peritoneal dialysis can be added from the concentrate source **104**. The concentrates in the concentrate source **104** are utilized to create a peritoneal dialysis fluid that matches a dialysis prescription. Concentrate pump **105** and concentrate valve **111** can control the movement of concentrates from the concentrate source **104** to the peritoneal dialysate generation flow path **101** in a controlled addition. Concentrate valve **111** can be replaced with a hose T. A hose T is a fluid connector in a T-shape, with a port at each end for fluid to enter or exit the hose T. The concentrates added from the concentrate source **104** to the peritoneal dialysate generation flow path **101** can include any component prescribed for use in peritoneal dialysate. Table 1 provides non-limiting exemplary ranges of commonly used components of peritoneal dialysate.

**Table 1**

| **Component** | **Concentration** |
|---|---|
| Sodium chloride | 132-134 mmol/L |
| Calcium chloride dehydrate | 1.25-1.75 mmol/L |
| Magnesium chloride hexahydrate | 0.25-0.75 mmol/L |
| Sodium Lactate | 35-40 mmol/L |
| Dextrose (D-glucose) monohydrate | 0.55-4.25g/dL |
| pH | 5-6 |
| Osmolality | 346-485 (hypertonic) |

To reduce the glucose degradation products (GDP) formed, some peritoneal dialysate systems use a low GDP formulation. Exemplary peritoneal dialysate concentrations for low GDP formulations are provided in Table 2. Generally, the low GDP peritoneal dialysate is provided in two separate bags, with one bag containing calcium chloride, magnesium chloride and glucose maintained at low pH, and the second bag containing sodium chloride and the buffer components, including sodium lactate and sodium bicarbonate. The two bags are mixed prior to use to generate a peritoneal dialysate with a neutral pH. Alternatively, a two-chamber bag can be used to prevent mixing of fluids prior to use wherein the chambers, can for example, be separated by a wall of a divider of any type.

**Table 2**

| Low GDP peritoneal dialysate formulations | |
|---|---|
| **Component** | **Concentration** |
| Sodium | 132-134 mEq/L |
| Calcium | 2.5-3.5 mEq/L |
| Magnesium | 0.5-1.0 mEq/L |
| Lactate | 0-40 mEq/L |
| Bicarbonate | 0-34 mEq/L |
| pH | 6.3-7.4 |
| % glucose (g/dL) | 1.5-4.25 |

One of skill in the art will understand that other components can be used in place of the components listed in Tables 1-2. For example, dextrose as listed in Table 1 is commonly used as an osmotic agent. However, other osmotic agents can be used in addition to, or in place of, the dextrose, including glucose, icodextrin or amino acids, including dialysate with multiple osmotic agents. Although the sources of sodium, calcium, and magnesium listed in Table 1 are chloride salts, other sodium, magnesium, and calcium salts can be used, such as lactate or acetate salts. Peritoneal dialysate may also contain buffers for maintaining pH of the peritoneal dialysate, including bicarbonate buffer, acetate buffer, or lactate buffer. Although not generally used in peritoneal dialysis, potassium chloride can be used for hypokalemic patients who don't receive sufficient potassium through diet. The concentrate source **104** can contain one or more osmotic agents, as well as one or more ion concentrates, such as concentrated sodium chloride, sodium lactate, magnesium chloride, calcium chloride, and/or sodium bicarbonate. The concentrate source **104** can be a single source of concentrates, including both osmotic agents and ion concentrates, or can include multiple sources of concentrates, with separate sources for the osmotic agents and ion concentrates. The system can have a single concentrate that has all components mixed for a daytime or overnight treatment for use in a home by a single patient. Alternatively, the concentrate source **104** can include separate sources for any solutes to be used in the peritoneal dialysate each with a separate concentrate pump to add each solute. Concentrate pump **105** pumps concentrated solutions from the concentrate source or sources **104** to the peritoneal dialysate generation flow path **101** in a controlled addition. Where more than one concentrate source is used, separate concentrate pumps can move each of the concentrates into the peritoneal dialysate generation flow path **101**, or a single concentrate pump can be used, with valves configured allow individual control over the movement of each of the concentrate solutions to the peritoneal dialysate generation flow path **101**.

After addition of solutes from the concentrate source **104**, the fluid in the peritoneal dialysate generation flow path **101** can contain all the necessary solutes for peritoneal dialysis. The peritoneal dialysate should reach a level of sterility for peritoneal dialysis. The level of sterility can be any level that meets an applicable regulatory requirement, such as a sterility assurance level of 10⁻⁶ required by the FDA, meaning that the chance a viable organism is present after sterilization is 1 in 1,000,000. The system can pump the fluid to a sterilization module for sterilization of the peritoneal dialysate. As shown in FIG. 1, the sterilization module can include one or more of a first ultrafilter **107**, a second ultrafilter **109**, and an optional UV light source **106**. The sterilization module can be any component or set of components capable of sterilizing the peritoneal dialysate. The sterilization module can be comprised of single or multiple ultrafilters. The number of ultrafilters can vary from one, two, three, four, and more depending on configuration and usage. A secondary component, such as a UV light source **106** or microbial filter (not shown), can be used in the sterilization module to provide additional sterilization of the peritoneal dialysate. The sterilization module can also include at least two ultrafilters, including second ultrafilter **109** for further sterilization of the fluid and redundancy of the system to protect against sterilization failure. The UV light source **106** can be positioned at any location in the peritoneal dialysate generation flow path **101**, including upstream of ultrafilter **107**, between ultrafilters **107** and **109** or downstream of ultrafilter **109**. The ultrafilters **107** and **109** used in the sterilization module can be replaced as necessary. In one non-limiting embodiment, the ultrafilters **107** and **109** can have a 3-6 month lifetime before replacement. However, no limitation on the lifespan of the ultrafilters is imposed by the system. The ultrafilters **107** and **109** can be any ultrafilter known in the art capable of sterilizing the peritoneal dialysate. A non-limiting example of an ultrafilter is the hollow fiber ForClean ultrafilter, available from Bellco, Mirandola (MO), Italy. In certain embodiments, the sterilization module **106** can use heat sterilization. The sterilization module can include a heater (not shown) to heat the prepared dialysate. Alternatively or additionally, the sterilization module can include a flash pasteurization module (not shown) to sterilize the dialysate through flash pasteurization. The sterilization module can include both heat-based sterilization components and filtration based sterilization components, with a processor, controller, or user adjusting the mode of sterilization based on the mode of use. For example, a heat based sterilization can be used when the peritoneal dialysate is generated for later use, while a filtration based sterilization can be used when the peritoneal dialysate is generated for immediate use.

The generated peritoneal dialysate can be pumped directly to an integrated cycler **110** for immediate infusion into a patient **134**. Alternatively, the dialysate can be pumped to an optional dialysate container **114** as a pre-prepared bolus of solution for storage until ready for use by a patient **134**. Valve **116** can control the movement of fluid to either the integrated cycler **110** or the dialysate container **114**. Stored dialysate in dialysate container **114** can be pumped as needed to the integrated cycler **110** by pump **115** through valve **117**. The dialysate container **114** can include one or more sterilized dialysate bags. The dialysate bags, once filled with peritoneal dialysate, can be stored until needed by the patient **134**. The dialysate container **114** can alternatively be a reusable sterilized container or bag. The reusable container or bag can be cleaned and sterilized daily, or at set time periods. Alternatively, the dialysate container **114** can be any type of storage container, such as a stainless-steel container. The dialysate container **114** can store enough peritoneal dialysate for a single infusion cycle of peritoneal dialysate into the patient **134**, or enough peritoneal dialysate for multiple infusions into a patient **134**. Additional or alternative storage containers can be included at other locations in the peritoneal dialysate generation flow path **101**. A storage container can be included upstream of the sterilization module, and downstream of the water purification module **103**. Before the fluid is utilized in the cycler stage, the fluid can be pumped through the sterilization module to ensure sterility of stored fluid. Further, concentrates can be added to fluid before storing the fluid, or after storage of the fluid but prior to sterilization in the sterilization module.

The storage containers can be either upstream or downstream of the concentrate source **104**. The addition of concentrates to the fluid can happen either before storage of the fluid, or after storage of the fluid just before sterilization in the sterilization module.

By generating and immediately using the peritoneal dialysate, the dialysate storage time can be reduced, reducing the possibility of bacterial growth. A user interface can be included on the peritoneal dialysis generation machine in communication with the control system, allowing a patient **134** to direct the generation of peritoneal dialysate at a selected time as needed. Additionally, or alternatively, the peritoneal dialysate machine can include a timer, and the timer can cause the peritoneal dialysate machine to generate peritoneal dialysate at predetermined times according to the patient's **134** peritoneal dialysis schedule. Alternatively, the peritoneal dialysate generation machine can be equipped with wireless communication, such as Wi-Fi, Bluetooth, Ethernet, or any other wireless communication system known in the art. The user can direct the peritoneal dialysis machine to generate peritoneal dialysate at a specified time from any location. By using a timer, user interface, or wireless communication to control the generation of peritoneal dialysate on demand, the peritoneal dialysate storage time can be reduced, lowering the chances of generating significant amounts of degradation products or allowing bacterial growth.

The peritoneal dialysate can be generated and used in real time, with direct infusion of the peritoneal dialysate into the patient **134** through the integrated cycler **110**. For real time generation and use of the peritoneal dialysate, the flow rate of fluid through the peritoneal dialysate generation flow path **101** can be between 50 and 300 ml/min. With the online generation of fluid described, a flow rate of 300 ml/min can support an exchange time of between 10 and 15 minutes for a full cycle of draining and filling the peritoneal cavity of a patient **134**. If a dialysate container **114** is used to store generated peritoneal dialysate, the flow rate of fluid through the peritoneal dialysate generation flow path **101** can be any flow rate capable of producing the necessary peritoneal dialysate. In certain embodiments, the flow rate can be at least around 15 mL/min, which will produce 20 L of peritoneal dialysate in 24 hrs. The integrated cycler **110** can then infuse the generated peritoneal dialysate into the peritoneal cavity of a patient **134**. The integrated cycler **110** and the rest of the system can communicate for the purposes of generation and use of the peritoneal dialysate by any method known in the art, including Bluetooth, Wi-Fi, Ethernet, or direct hardware connections to meet patient or clinic needs. Additional valves and regulators (not shown in FIG. 1) can be included to aid in connection and operation of the peritoneal dialysate generation flow path **101** and integrated cycler **110**. The integrated cycler **110** and the peritoneal dialysate generation flow path **101** can communicate directly, or can each communicate with a control system for control over the generation and use of the peritoneal dialysate.

In certain embodiments, the dialysate container **114** can store enough peritoneal dialysate for multiple infusions into the patient **134**, including enough peritoneal dialysate for one day or more of treatment. A timer can be included in the control system and can cause the machine to generate fresh peritoneal dialysate each day or at set times.

The integrated cycler **110** can include a metering pump **119** for metering peritoneal dialysate into the peritoneal cavity of the patient **134**. An in-line heater **118** heats the peritoneal dialysate to a desired temperature prior to infusion into the patient **134**. A pressure regulator **120** ensures the peritoneal dialysate pressure is within a predetermined range safe and comfortable for infusion into the patient **134**. The metering pump **119** can use any safe pressure for infusing fluid into the patient **134**. Generally, the pump pressures are on average set at ±10.3 kPa or 77.6 mmHg. If there is no fluid flow, the maximum pressure can increase to ±15.2 kPa or 113.8 mmHg for a short period, such as less than 10 seconds. The peritoneal dialysate is infused into the peritoneal cavity of the patient **134** through infusion line **124**. An additional microbial filter (not shown) may be used to sterilize the peritoneal dialysis fluid immediately before the peritoneal dialysate enters the patient **134**. After a dwell period, the peritoneal dialysate is drained from the patient **134** through drain line **123**. Pump **122** provides a driving force for removing the peritoneal dialysate from the patient **134**. Treatment, other than the first full cycle for a patient in APD, generally begins with drainage of the peritoneal cavity of the patient **134**, prior to infusing the fresh peritoneal dialysate into the patient **134**. An optional waste reservoir **121** can be included to store the used peritoneal dialysate for disposal. Alternatively, the drain line **123** can be directly connected to a drain for direct disposal. A standard waste reservoir **121** is 15 L, however, the waste reservoir **121** can be any size, including between around 12 and around 20 L. For patients requiring a higher drainage, a drain manifold can be included for connecting multiple waste reservoirs. There is no set rate for draining of peritoneal dialysate from the peritoneal cavity of the patient **134**, and any flow rate can be used with the integrated cycler **110**.

Various sensors positioned in the peritoneal dialysate generation and infusion system ensure that the generated fluid is within predetermined parameters. Flow meter **135** ensures the incoming water is at a correct flow rate, while pressure sensor **136** ensures the incoming water is at an appropriate pressure. Conductivity sensor **125** is used to ensure that the water exiting water purification module **103** has been purified to a level safe for use in peritoneal dialysis. Conductivity sensor **126** ensures the conductivity of the dialysate after the addition of concentrates from concentrate source **104** is within a predetermined range. Refractive index sensor **127** ensures that the concentration of the osmotic agents is within a predetermined range. pH sensor **128** ensures the pH of the peritoneal dialysate is within a predetermined range. After passing through the sterilization module including second ultrafilter **109**, pH sensor **129** and conductivity sensor **130** are used to ensure that no changes in the pH or conductivity have occurred during purification or storage of the dialysate in dialysate container **114**. The integrated cycler **110** has flow meter **131**, pressure sensor **132** and temperature sensor **133** to ensure that the dialysate being infused into the patient **134** is within a proper flow rate, pressure, and temperature range. The flow meter **131** can also calculate the volume of solution infused into the patient **134**. The pressure sensor **132** can monitor the pressure in the peritoneal cavity.

Overfill, or excessive solution in the peritoneal cavity beyond the target volume may present complications in therapy. Overfill can be caused by many factors, including failing to fully drain the peritoneal cavity prior to infusion of fresh peritoneal dialysate. In any embodiment, the integrated cycler **110** can start therapy with a drain step to ensure that no peritoneal dialysate remains in the peritoneal cavity. Monitoring both pressure and volume of peritoneal dialysate introduced to the patient **134** can avoid overfill. If the pressure rises to a certain point, the system can be programmed to end filling or send an alert to a user to complete filling of the peritoneal cavity at a desired level. The volume of peritoneal dialysate extracted from and introduced to the patient **134** can also be monitored with flow meters to ensure proper volumes of exchanges. Draining the peritoneal cavity can be performed in a similar manner by monitoring the pressure and volume of the drained peritoneal dialysate.

As illustrated in FIG. 1, the necessary solutes can be added to the peritoneal dialysate generation flow path **101** from a single concentrate source **104**. The solutes can be present in concentrated from within the concentrate source **104** in a fixed ratio for peritoneal dialysis, as shown in Table 1. Using a single concentrate source **104** for all solutes results in peritoneal dialysate having a fixed ratio of each of the solutes.

Table 3 provides exemplary non-limiting ranges of solutes that can be added from a single concentrate source **104** to the peritoneal dialysate generation flow path **101**, including the starting concentration of the solutes in the concentrate source, as well as exemplary final volumes of the solutes in the dialysate and the exemplary flow rates of both the solutes and the water in the peritoneal dialysate generation flow path **101** that will achieve those concentrations. The solutes shown in Table 3 are traditional peritoneal dialysate solutes. Table 4 shows exemplary ranges of solutes that can be used as a low GDP formulation. Table 5 shows exemplary ranges of solutes that can be used with icodextrin as the osmotic agent. Icodextrin is sometimes used as an osmotic agent for a long dwell period. If dextrose or glucose is used in a long dwell period, reabsorption of the ultrafiltrate can occur, reducing the net volume of fluid removed. Icodextrin can result in a long sustained ultrafiltration, and can provide improved ultrafiltration efficiency over a long dwell period. One of skill in the art will understand that the concentrations of any of the solutes shown in Tables 3-5 can be altered by altering the flow rates of the system pump **108** or concentrate pump **105**. However, the ratio of the solutes included is fixed if using a single concentrate source **104**. If the ratio of the solutes needs to be altered for any reason, a new concentrate solution may be needed.

**Table 3**

| Exemplary solutes for addition from a single concentrate source | | | |
|---|---|---|---|
| **Component** | **Concentration (g/l)** | **Solution volume (ml/L)** | **Flow rate (ml/min)** |
| Glucose | 100 - 850 | 50-400 | 1 - 18 |
| Sodium Chloride | 13 - 108 | 50-400 | 1 - 18 |
| Sodium Lactate | 11 - 90 | 50-400 | 1 - 18 |
| MgCl₂.6H₂O | 0.13 - 1.02 | 50-400 | 1 - 18 |
| CaCl₂.2H₂O | 0.6 - 5.1 | 50-400 | 1 - 18 |
| Water | | 600-950 | 50-1000 |

**Table 4**

| Exemplary solute ranges in a low GDP solution | | | |
|---|---|---|---|
| **Component** | **Concentration (g/l)** | **Solution volume (ml/L)** | **Flow rate (ml/min)** |
| Glucose | 100 - 900 | 50-400 | 1 - 18 |
| Sodium Chloride | 13 - 108 | 50-400 | 1 - 18 |
| Sodium Lactate | 11 - 90 | 50-400 | 1 - 18 |
| MgCl₂.6H₂O | 0.13 - 1.02 | 50-400 | 1 - 18 |
| CaCl₂.2H₂O | 0.6 - 5.1 | 50-400 | 1 - 18 |
| Water | | 600-950 | 50-1000 |

**Table 5**

| Exemplary solute ranges in icodextrin solution | | | |
|---|---|---|---|
| **Component** | **Concentration (g/l)** | **Solution volume (ml/L)** | **Flow rate (ml/min)** |
| Icodextrin | 100 - 850 | 100-400 | 2-37 |
| Sodium Chloride | 13 - 108 | 100-400 | 1 - 18 |
| Sodium Lactate | 11 - 90 | 100-400 | 2-37 |
| MgCl₂.6H₂O | 0.13 - 1.02 | 100-400 | 2-37 |
| CaCl₂.2H₂O | 0.6 - 5.1 | 100-400 | 2-37 |
| Water | | 600-900 | 50-1000 |

Although using a single concentrate source **104** in the system requires a fixed ratio of solutes in the generated peritoneal dialysate, a single concentrate source **104** provides certain advantages. Storage requirements are decreased, as only a single concentrate solution needs to be stored for a given dialysate prescription. There is also a lower risk of patient error in adding solutes to the dialysate in the proper amounts. A single concentrate source **104** also requires less supplies, less pumps, and less hardware. Further, because fewer containers are needed, the containers are easier to manage, clean, and disinfect. A higher concentration of solutes in the concentrate source **104** will allow minimization of the container size and maximization of the source water used in PD solution preparation, lowering costs. The limiting factor is mutual solubility of the components, which is generally limited by glucose or icodextrin solubility. The flow rate for the source water can be optimized to adjust the time required to prepare the solution. In the case of on-demand dialysate preparation, a high flow rate is desired to minimize the time needed to prepare the solution. The flow rate limit will be controlled by the metering accuracy of the concentrate pump **105** at the rate required to match the water feed. With a single concentrate source **104**, about 150 ml/exchange can be needed, which corresponds to about 600 ml/day or 4.2 L/week. The concentrate source **104** can be sized depending on the needs of the user, with a larger concentrate source requiring less frequent refilling.

The system can also include an additional waste reservoir (not shown in FIG. 1) to collect any waste fluid generated by the water purification module **103** or other components. Alternatively, waste reservoir **121** can also be used to collect any waste fluid generated by the water purification module **103** or other components. The waste reservoir **121** collects effluent generated during disinfection and/or effluent generated by the purification modules, such as a reverse osmosis system.

The peritoneal dialysate generation flow path **101** and integrated cycler **110** can be disinfected with a disinfection solution through on-board disinfection if the components of the peritoneal dialysate generation flow path **101** and integrated cycler **110** are to be reused. Disinfection may not be required with a fully disposable peritoneal dialysate generation flow path **101**. The peritoneal dialysate generation flow path **101** and integrated cycler **110** can be configured to form a loop by connecting the portion of the peritoneal dialysate generation flow path **101** that connects to water tank **102** or the direct connection **112** to a water source to the infusion line **124**. The disinfection solution can be introduced into the peritoneal dialysate generation flow path **101** and recirculated through the fluid lines by system pumps **108** and **119.** Alternatively, the peritoneal dialysate generation flow path **101** and integrated cycler **110** can be disinfected separately after disconnection of the integrated cycler **110** from the peritoneal dialysate generation flow path **101**. The disinfection solution can be a citric acid solution, a peracetic acid solution, a bleach solution, or any other disinfection solution known in the art. Disinfectant can be circulated through the flow loop and heated. The disinfectant can be heated to any temperature capable of disinfecting the system, including temperatures of at least 80 °C or greater. The disinfectant can be introduced to the flow loop and recirculated at elevated temperatures to ensure complete disinfection.

Solutes can be added to the peritoneal dialysate generation flow path **201** from two or more separate concentrate sources, as shown in FIG. 2. The peritoneal dialysate generation flow path **201** can be fluidly connected to a water source and a water purification module upstream of the concentrate sources **202-206**, and a sterilization module, an integrated cycler, and optionally a dialysate container downstream of the concentrate sources **202-206**, as illustrated in FIG. 1. For clarity, these components have been omitted from FIG. 2.

As illustrated in FIG. 2, the concentrate sources **202-206** can include one or more ion concentrate sources, such as sodium chloride source **202** containing sodium chloride to be added in a controlled addition to the peritoneal dialysate generation flow path **201** by concentrate pump **207** through valve **212**, sodium lactate source **203** containing sodium lactate to be added in a controlled addition to the peritoneal dialysate generation flow path **201** by concentrate pump **208** through valve **213**, magnesium chloride source **204** containing magnesium chloride to be added in a controlled addition to the peritoneal dialysate generation flow path **201** by concentrate pump **209** through valve **214**, and calcium chloride source **205** containing calcium chloride to be added in a controlled addition to the peritoneal dialysate generation flow path **201** by concentrate pump **210** through valve **215**. One of skill in the art will understand that other ions can be used in formulation of peritoneal dialysate, and each can be contained in a separate ion concentrate source or combined into one or more combined ion concentrate sources. The concentrate source also includes one or more osmotic agent sources, such as dextrose source **206** containing dextrose to be added to the peritoneal dialysate generation flow path **201** by concentrate pump **211** through valve **216**. Any of the concentrate pumps can include flow meters to control the addition of the solutes. A glucose source and/or an icodextrin source can be used in addition to, or in place of, dextrose source **206**. Multiple osmotic agents can be added to the peritoneal dialysate generation flow path **201** from one or more osmotic agent sources. One of skill in the art will understand other solutes can be used alternatively to, or in addition to, the solutes illustrated in FIG. 2. A control system in electronic communication with each of the concentrate pumps can control the movement of fluid from the concentrate sources to the peritoneal dialysate generation flow path **201**. The amount of each of the concentrates moved into the peritoneal dialysate generation flow path **201** can be controlled to result in peritoneal dialysate having a prescribed solute concentration, as determined by a doctor or health care provider. The valves **212-216** can optionally be replaced with hose T junctions with additional components for preventing backflow into the concentrate source line if that particular line is not being used. Optional sensors **217**, **218**, **219**, and **220** ensure the solute concentration in the dialysate is at the correct level after each addition. The sensors **217-220** can be any type of sensor appropriate to confirm delivery of the concentrate, such as conductivity sensors. Optional pH sensor **221** can be used to ensure that the pH is a proper level after addition of sodium lactate or other buffer. Optional refractive index meter **222** ensures the dextrose concentration in the dialysate is at the prescribed level. An additional sensor can be included upstream of sodium chloride source **202** for sensing the conductivity of the water prior to addition of concentrates. One of skill in the art will understand that additional sensor arrangements can be used in the described system. Any number of sensors can be included to monitor the peritoneal dialysate concentration, including 1, 2, 3, 4, 5, 6, 7, or more sensors. The concentrate sources can contain the solutes in either solid, powdered, or solution form. A solid or powdered source of solutes can be dissolved by the system by drawing fluid from the peritoneal dialysate generation flow path **201** into the concentrate source to generate a solution with a known concentration, such as a saturated solution of the solutes. During the process of dissolution of the solutes, mechanical, vibration, heating, or other forms of assistance may be used to dissolve the solid or powder solutes. The resulting solution is added to the peritoneal dialysate generation flow path as explained. Although shown as a refractive index meter **222** in FIG. 2, one of skill in the art will understand that alternative methods of measuring the osmotic agent concentration can be used, including enzyme based sensors or pulsed amperometric detection.

Although illustrated as a single concentrate source in FIG. 1, and five separate concentrate sources in FIG. 2, one of skill in the art will understand that any number of concentrate sources can generate the peritoneal dialysate, including 1, 2, 3, 4, 5, 6, 7, or more concentrate sources. Any two or more of the separate concentrate sources illustrated in FIG. 2 can be combined into a single solute source, such as by combining all or some of the ion concentrate sources into a single ion concentrate source where the mixed contents do not cause precipitation of the mixed concentrates. Although each concentrate source is illustrated in FIG. 2 with a separate concentrate pump and fluid line, one of skill in the art will understand that more than one concentrate source can use a single pump and fluid line, with valves arranged thereon for controlled addition to the peritoneal dialysate generation flow path **201**.

The concentrate sources **202-206** can be single use concentrate sources or disposable concentrate sources. The disposable concentrate sources are used in a single peritoneal dialysate generation process and then disposed. Multiple use concentrate sources are used repeatedly, and refilled as necessary with the solute.

Table 6 provides exemplary, non-limiting, ranges of solutes that can be added to the peritoneal dialysate using a separate osmotic agent source, glucose in Table 6, and a separate ion concentrate source containing sodium chloride, sodium lactate, magnesium chloride, calcium chloride and sodium bicarbonate. Because the glucose is added separately from the ion concentrates, the ratio of glucose to the other solutes can be varied depending on the needs of the patient.

**Table 6**

| Exemplary ranges of solutes in a two-concentrate source system | | | |
|---|---|---|---|
| **Component** | **Concentration (g/l)** | **Solution volume (ml/L)** | **Dialysate composition** |
| **Part A** | | | |
| Glucose | 850 | 6 - 53 | 0.55-4.5 g/dL |
| **Part B** | | | |
| NaCl | 269 | 20 | 92 mmol/L |
| Sodium Lactate | 84 | 20 | 15 mmol/L |
| MgCl₂.6H₂O | 5 | 20 | 0.5 mmol/L |
| CaCl₂.2H₂O | 18 | 20 | 2.5 mmol/L |
| NaHCO₃ | 105 | 20 | 25 mmol/L |
| Water | | 927-979 | 56.10 |

By using multiple concentrate sources, greater individualization and therapy customization can be achieved for each patient. With a single concentrate source, all solutes in the generated peritoneal dialysate must be present in a fixed ratio. By using more than one concentrate source, the ratio of solutes used in the peritoneal dialysate can be altered as the concentration of each of the osmotic agent and ion solutes can be individually controlled. For example, as illustrated by Table 6, with a single ion concentrate source and a single osmotic agent source, peritoneal dialysate with greater or less osmotic agent per concentration of ions can be generated, providing the ability to adjust the tonicity of the peritoneal dialysate solution independently of the electrolyte composition to meet the UF needs of any patient with a single set of solutions and allowing greater control over ultrafiltration. The ultrafiltration rate that results from using the peritoneal dialysate solutions can be altered by altering the concentration of the osmotic agent independently of the ionic solutes, or by changing the osmotic agent used. Because the system is not limited to discrete glucose or other osmotic agent concentrations like known commercial solutions; the system can customize the peritoneal dialysate solutions to meet the ultrafiltration needs of patient as determined by a healthcare provider. As illustrated in Table 6, the glucose level in the peritoneal dialysate solution can be varied from 0.55 g/dL to 4.5 g/dL, while maintaining the electrolytes and buffer components constant, allowing the system to cover the range of glucose formulations currently offered commercially using a single Part A and Part B composition.

In certain embodiments, two osmotic agent sources can be used, such as a dextrose source and an icodextrin source. With two osmotic agent sources, one could use dextrose during the daytime exchanges for CAPD and icodextrin during the night dwell to take advantage of the higher UF removal from icodextrin. Conversely, dextrose could be used during the night dwell and icodextrin for the extended daytime dwell in APD systems.

By using separate concentrate sources for each solute, complete individualization of the concentrations and ratios of solutes in the peritoneal dialysate can be achieved. Table 7 provides exemplary ranges of solutes that can be used in peritoneal dialysate as made by a system with each solute in a separate concentrate source. An advantage of using separate concentrate sources for each solute is that virtually any peritoneal dialysate solution composition can be prepared from a single set of component formulations. A system with separate concentrate sources for each solute is useful for patients whose prescriptions change periodically due to diet or other factors. Such patients would need to store multiple formulations if using only one or two concentrate sources, and the risk of errors would be increased.

**Table 7**

| Exemplary dialysate composition from a multi-source system | | | |
|---|---|---|---|
| **Component** | **Concentration (g/l)** | **Solution volume (ml/L)** | **Dialysate composition** |
| Part A: Glucose | 850 | 6 - 53 | 0.55-4.5 g/dL |
| Part B: NaCl | 320 | 15-18 | 132-134 mmol/L |
| Part C: Na Lactate | 1000 | 2-4 | 15-40 mmol/L |
| Part D: MgCl2.6H2O | 500 | 0.2-0.4 | 0.5 -1.0 mmol/L |
| Part E: CaCl2.2H2O | 700 | 0.5-1.0 | 2.5-3.5 mmol/L |
| Part F: NaHCO3 | 85 | 0-34 | 0-34 mmol/L |
| Part G: Icodextrin | 1000 | 0-75 | 0-7.5g/dL |
| Water | | 820-971 | |

The one or more concentrate sources can be detachable from the rest of the system for sterilization. The concentrate sources can also be sterilized each time the concentrate sources are filled with new concentrate solutions. Further, the concentrate sources can be sterilized after a set number of uses, or after a set period of time. Moreover, the concentrate sources and the remaining components including the fluid lines of the peritoneal dialysate generation system can be sterilized without any of the components by passing a disinfection solution, such as a citric acid, peracetic acid, or bleach solution, through all of the lines and containers of the system.

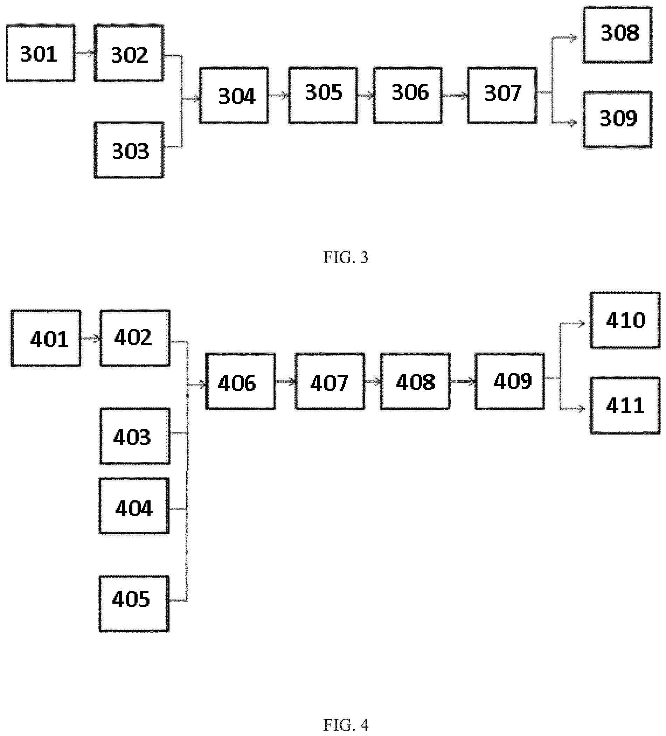

FIG. 3 illustrates an overview of generating peritoneal dialysate in accordance with any embodiment of the invention. Water from a water source **301** can be purified by a water purification module **302**, as explained. Concentrates from a single concentrate source **303**, which can contain both ion concentrates and one or more osmotic agents, can be added to the purified water to generate a non-sterile peritoneal dialysate solution **304**. The non-sterile peritoneal dialysate solution **304** is sterilized by a sterilization module **305**, which may include an ultrafilter (not shown). As explained, the peritoneal dialysate can be further purified by additional components in the sterilization module **306**, such as by ultrafiltration with a second ultrafilter, by a microbial filter, or by an optional UV light source, to generate a sterilized peritoneal dialysate **307**. The sterilized peritoneal dialysate **307** can be stored or used by any method described herein, including by immediately infusing the peritoneal dialysate into a patient **308**, or dispensing the peritoneal dialysate into a dialysate container for later use in peritoneal dialysis **309**, as illustrated in FIG. 1.

FIG. 4 illustrates an overview of generating peritoneal dialysate with multiple concentrate sources. Water from a water source **401** can be purified by a water purification module **402**, as explained. Concentrates from an ion concentrate source **403**, which can contain sodium, magnesium, calcium, and bicarbonate, as well as any other ions to be used in peritoneal dialysis, can be added to the purified fluid. An osmotic agent, such as dextrose, can be added from a first osmotic agent concentrate source **404**. A second osmotic agent, such as icodextrin, can be added from a second osmotic agent concentrate source **405**. As illustrated in FIG. 2, any number of concentrate sources can be used for further individualization of the peritoneal dialysate, including separate sources for each of the ions used. After addition of the ion and osmotic agent concentrates, the fluid contains all necessary components for use in peritoneal dialysis as non-sterilized peritoneal dialysate **406**. The non-sterile peritoneal dialysate **406** can be sterilized by a sterilization module **407**, which can include an ultrafilter or other sterilization components. The peritoneal dialysate can be further sterilized by the sterilization module **408**, either by ultrafiltration with a second ultrafilter, a microbial filter, or further sterilized with an optional UV light source, to generate a sterilized peritoneal dialysate **409**. The sterilized peritoneal dialysate **409** can be stored or used by any method described herein, including by immediately infusing the peritoneal dialysate into a patient **410,** or dispensing the peritoneal dialysate into a dialysate container for later use in peritoneal dialysis **411**, as illustrated in FIG. 1.

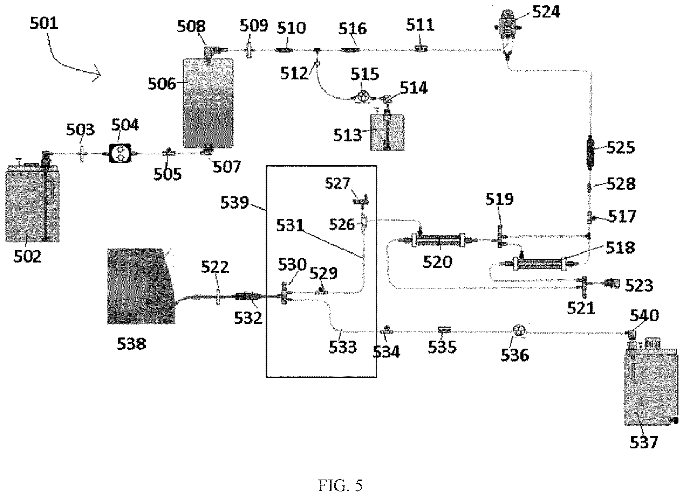

FIG. 5 illustrates an alternative peritoneal dialysate generation flow path **501** with an integrated cycler **539**. Water from a water source **502** can be pumped through filter **503** by system pump **504**. The filter **503** can remove any particulate matter from the water prior to entering the peritoneal dialysate generation flow path **501**. The water is then pumped through a water purification module, illustrated as a sorbent cartridge **506** in FIG. 5. As described, the water purification module can alternatively or additionally include activated carbon, a reverse osmosis module, a carbon filter, an ion exchange resin, and/or a nanofilter. The water enters the sorbent cartridge **506** through sorbent cartridge inlet **507** and exits through sorbent cartridge outlet **508**. Pressure sensor **505** measures the pressure across sorbent cartridge **506**. Filter **509** removes any particulate matter in the fluid after exiting sorbent cartridge **506**. A conductivity sensor **510** determines the conductivity of the fluid exiting sorbent cartridge **506** to ensure the water has been purified. To generate the peritoneal dialysate, concentrates are added from concentrate source **513** through concentrate connector **514** by concentrate pump **515**. Although shown as a single concentrate source **513** in FIG. 5, concentrates can be added from any number of separate concentrate sources. Concentrate filter **512** removes any particulate matter from the concentrate before entering the peritoneal dialysate generation flow path **501**. A conductivity sensor **516** determines the conductivity of the generated peritoneal dialysate after addition of the concentrates to ensure the peritoneal dialysate has the correct solute concentrations. Flow sensor **511** determines the flow rate of the fluid after addition of the concentrates. pH sensor **524** determines the pH of the peritoneal dialysate to ensure the peritoneal dialysate has a proper pH. The peritoneal dialysate can be heated to a desired temperature by heater **525**. Temperature sensor **528** ensures the peritoneal dialysate is heated to an appropriate temperature before infusion into the patient **538**. The heater **525** can be placed at any location in the flow path prior to delivery to the patient **538**. In any embodiment, the heater **525** can be located after the exit of the sterilization module, particularly if fluid is stored prior to passing through the sterilization module. The desired temperature of the peritoneal dialysate can be between around 20°C to around 41°C. As used herein, around 20°C can include between 19.0 °C and 21.0 °C, and around 41°C can include between 39.0 °C and 41.0°C, or similar as understood by those of skill in the art. In certain embodiments, the desired temperature can be between around 25°C to around 40°C, around 36.5°C to around 37.25°C, around 25°C to around 35°C, or around 30°C to around 40°C. In a preferred embodiment, the desired temperature can be 37 ± 2 °C.

As described, the peritoneal dialysate is sterilized by pumping the peritoneal dialysate through a sterilization module, which can include first ultrafilter **518**, and optionally a second ultrafilter **520** and/or an optional UV light source (not shown). Pressure sensor **517** measures the fluid pressure prior to the fluid entering the sterilization module, shown as ultrafilters **518** and **520**, and is used in the control circuit to control the pressure. The fluid passes through first ultrafilter **518**, through valve **519**, and then through second ultrafilter **520**. Connector **523**, three way valve **521**, and valve **519** allow backflushing and disinfection of the ultrafilters **518** and **520**. The fluid is then pumped into the integrated cycler **539** for use in peritoneal dialysis. As described, the system can include a dialysate container (not shown) for storage of the generated peritoneal dialysate until used by the patient **538** at any location, including upstream or downstream of the sterilization module.

The integrated cycler **539** includes an infusion line **531** and a drain line **533**. Bubble trap **526** traps air bubbles present in the heated dialysate. The air is vented from the system through bubble trap valve **527**. Pressure sensor **529** ensures the pressure of the fluid is within a predetermined range. In certain embodiments, the predetermined range can be a pressure of between -200 mmHg to 500 mmHg, from -50 mmHg to 100 mmHg, from 0 mmHg to 100 mmHg, from -50 mmHg to 200 mm Hg, from 200 mmHg to 500 mmHg, or from 100 mmHg to 400 mmHg. The infusion line **531** is connected to a three-way valve **530**, which controls fluid movement between the infusion line **531**, the patient **538**, and the drain line **533**. The three way valve **530** is connected through connector **532** to a catheter inserted into the peritoneal cavity of the patient **538**. A filter **522** can be included between the three-way valve **530** and the catheter for additional cleaning of the peritoneal dialysate prior to entering a patient **538**. In any embodiment, the filter **522** can be a disposable filter. The peritoneal dialysate is infused into the patient **538** and held for a dwell period. After the dwell period, the fluid is pumped out of the peritoneal cavity of the patient **538** by drain pump **536**. The three-way valve **530** is switched to direct fluid into the drain line **533**. Pressure sensor **534** measures the pressure of fluid in the drain line **531** to ensure proper drainage. Flow meter **535** measures the flow rate and volume of fluid removed from the patient **538**. The drain line **531** is connected to a drain or waste reservoir **537** through connector **540** for collection and disposal of the used peritoneal dialysate.

For automated disinfection of the system, connector **540** can be connected to connector **523** to form a flow loop. Disinfectant can be circulated through the flow loop and heated. The disinfectant can be heated to any temperature capable of disinfecting the system, including temperatures of at least around equal to 80 °C or greater (≥80) when using citric acid as a disinfectant. Peracetic acid or bleach can be used to disinfect the system at room temperature. The disinfectant can be introduced to the flow loop and recirculated at elevated temperatures to ensure complete disinfection. The disinfectant used can be any suitable disinfectant known in the art, including peracetic acid, citric acid, or bleach. The connectors and components of the system can be gamma and autoclave compatible to resist the high temperatures used during disinfection. The system can be primed by introducing a priming fluid to the peritoneal dialysate generation flow path **501** and integrated cycler **539**.

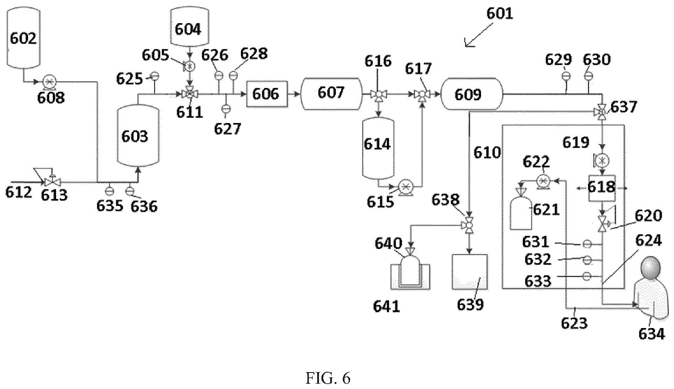

FIG. 6 illustrates an alternative embodiment of the system. Fluid from a water source, such as water tank **602,** can be pumped into the peritoneal dialysate generation flow path **601**. Additionally, or as an alternative to a water tank **602**, the system can use a direct connection to a water source **612**. System pump **608** can control the movement of fluid through the peritoneal dialysate generation flow path **601**. If a direct connection to a water source **612** is used, a pressure regulator **613** can ensure that an incoming water pressure is within a predetermined range. The system pumps the fluid from water source **602** or **612** through a water purification module **603** to remove chemical contaminants in the fluid in preparation for creating dialysate.

After the fluid passes through the water purification module **603**, the fluid is pumped to a concentrate source **604**, where necessary components for carrying out peritoneal dialysis can be added from the concentrate source **604**. The concentrates in the concentrate source **604** are utilized to create a peritoneal dialysis fluid that matches a dialysis prescription. Concentrate pump **605** and concentrate valve **611** can control the movement of concentrates from the concentrate source **604** to the peritoneal dialysate generation flow path **601** in a controlled addition. Alternatively, concentrate valve **611** can be a hose T or backflow restricting hose T. The concentrates added from the concentrate source **604** to the peritoneal dialysate generation flow path **601** can include components required for use in peritoneal dialysate. Upon addition of solutes from the concentrate source **604**, the fluid in the peritoneal dialysate generation flow path **601** can contain all the necessary solutes for peritoneal dialysis. The peritoneal dialysate should reach a level of sterility for peritoneal dialysis, as described. As shown in FIG. 6, the sterilization module can include one or more of a first ultrafilter **607**, a second ultrafilter **609**, and a UV light source **606**.

The generated peritoneal dialysate can be pumped directly to an integrated cycler **610** for immediate infusion into a patient **634**. Alternatively, the dialysate can be pumped to an optional dialysate container **614** as a pre-prepared bolus of solution for storage until ready for use by a patient **634**. Valve **616** can control the movement of fluid to either the dialysate container **614**. Stored dialysate in dialysate container **614** can be pumped as needed to back into the peritoneal dialysate generation flow path **601** by pump **615** through valve **617**. The dialysate container **614** can store enough peritoneal dialysate for a single infusion of peritoneal dialysate into the patient **634**, or enough peritoneal dialysate for multiple or continuous infusions into one or multiple patients.

The generated peritoneal dialysate can be pumped to valve **637**. Valve **637** can control movement of the peritoneal dialysate to any of three options. First, the peritoneal dialysate can be pumped to integrated cycler **610**, second diverted for use with a non-integrated external cycler **639**, or third diverted to a dialysate container **640**. All three options can be performed contemporaneously or selectively. If diverted to the non-integrated external cycler **639**, the peritoneal dialysate can be pumped via valve **638**. Valve **638** can control the movement of the peritoneal dialysate through either a direct connection to an external cycler **639** or to a dialysate container **640**. Alternative valve and pump configurations for performing the same functions are contemplated by the present invention. For example, the direct connection to an external cycler **639** can use any type of connector known in the art. The connectors can be single-use or reusable connectors and should provide for sterile transfer of fluids. The connectors should preferably be closed connectors, to avoid contact between the fluids and the external environment. A non-limiting example of a connector that can be used for a direct connection to an external cycler is the INTACT® connectors provided by Medinstill Development LLC, Delaware, US. The dialysate container **640** can be heated with an optional heater **641** and then used in peritoneal dialysis. The connectors to the dialysate container **640** can be any type of connector known in the art. The connectors can be single use or disposable connectors that provide transfer of sterile fluids. A non-limiting example of connectors that can be used with the described system is the Lynx®-Millipore connectors available from Merck KGaA, Darmstadt, Germany.

The integrated cycler **610** can include a metering pump **619** for metering peritoneal dialysate into the peritoneal cavity of the patient **634**. A heater **618** heats the peritoneal dialysate to a desired temperature prior to infusion into the patient **634**. A pressure regulator **620** ensures the peritoneal dialysate pressure is within a predetermined range safe for infusion into the patient **634**. The metering pump **619** can use any safe pressure for infusing fluid into the patient **634**. Generally, the pump pressures are on average set at ±10.3 kPa or 77.6 mmHg. If there is no fluid flow, the maximum pressure can increase to ±15.2 kPa or 113.8 mmHg for a short period, such as less than 10 seconds. The peritoneal dialysate is infused into the peritoneal cavity of the patient **634** through infusion line **624**. After a dwell period, the peritoneal dialysate is drained from the patient **634** through drain line **623**. Pump **622** provides a driving force for removing the peritoneal dialysate from the patient **634**. An optional waste reservoir **621** can be included to store the used peritoneal dialysate for disposal. Alternatively, the drain line **623** can be directly connected to a drain for direct disposal. The waste reservoir **621** can be any size, including between 12 and 25 L. For patients requiring a higher drainage, a drain manifold can be included for connecting multiple waste reservoirs.

Various sensors positioned in the peritoneal dialysate generation and infusion system ensure that the generated fluid is within predetermined parameters. Flow meter **635** ensures the incoming water is at a correct flow rate, while pressure sensor **636** ensures the incoming water is at an appropriate pressure. Conductivity sensor **625** is used to ensure that the water exiting water purification module **603** has been purified to a level safe for use in peritoneal dialysis. Conductivity sensor **626** ensures the conductivity of the dialysate after the addition of concentrates from concentrate source **604** is within a predetermined range. Refractive index sensor **627** insures that the concentration of the osmotic agents is within a predetermined range. pH sensor **628** ensures the pH of the peritoneal dialysate is within a predetermined range. After passing through the sterilization module including second ultrafilter **609**, pH sensor **629** and conductivity sensor **630** are used to ensure that no changes in the pH or conductivity have occurred during purification or storage of the dialysate in dialysate container **614**. The integrated cycler **610** has flow meter **631**, pressure sensor **632** and temperature sensor **633** to ensure that the dialysate being infused into the patient **634** is within a proper flow rate, pressure, and temperature range.

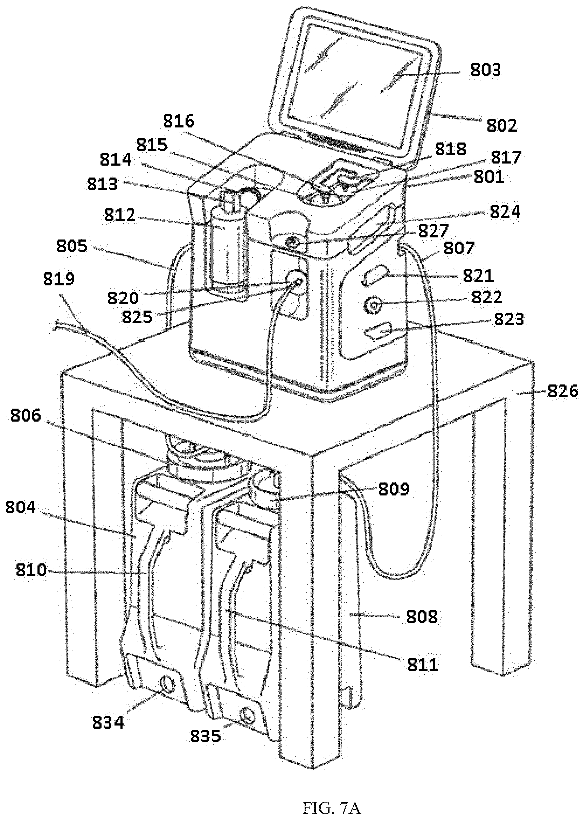

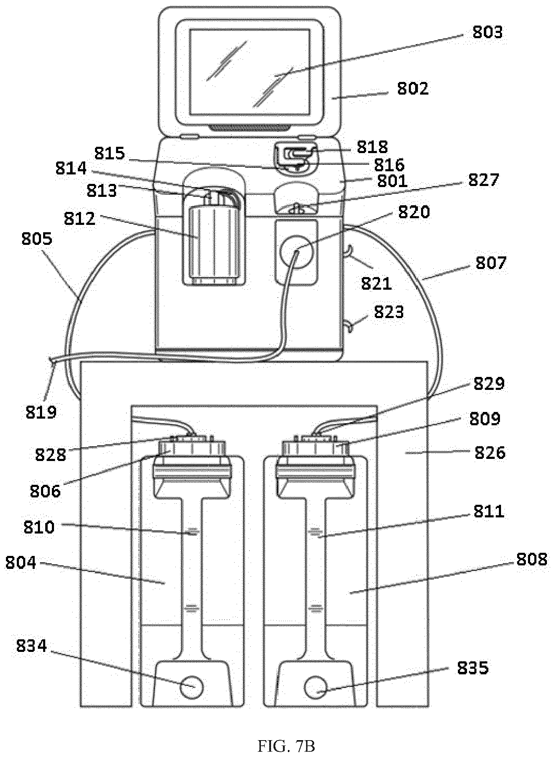

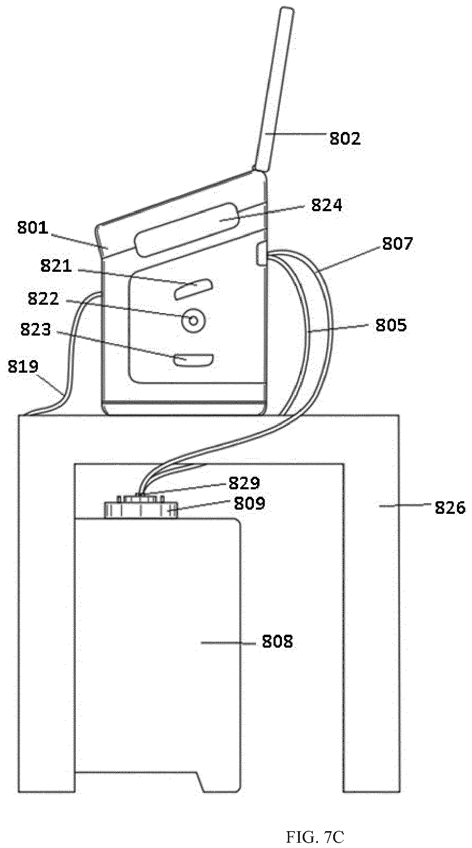

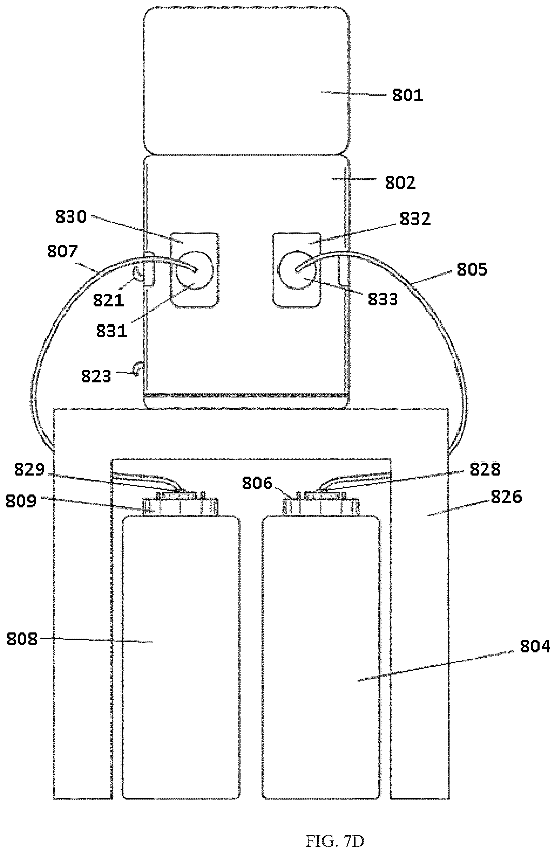

FIG.'s 7A-D illustrate a non-limiting embodiment of the peritoneal dialysate generation system arranged as a peritoneal dialysate generation cabinet **801**. FIG. 7A illustrates a perspective view of the peritoneal dialysate generation cabinet **801**, FIG. 7B illustrates a front view of the peritoneal dialysate generation cabinet **801**, FIG. 7C illustrates a side view of the peritoneal dialysate generation cabinet **801**, and FIG. 7D illustrates a back view of the peritoneal dialysate generation cabinet **801**.

A fluid line **805** can connect a water source **804** to the peritoneal dialysate generation cabinet **801**. The fluid line **805** can enter through a connector **828** in a top **806** of the water source **804**. The fluid line **805** connects to the peritoneal dialysate generation flow path as described with reference to FIG.'s 1 and 5-6 through a back of the peritoneal dialysate generation cabinet **801** through connector **832** having a fitting **833** for holding the fluid line **805**, as illustrated in FIG. 7D. Any of the fluid lines illustrated can be disconnected and removed from the system for cleaning and replacement. A pump (not shown) can provide a driving force for the movement of fluid throughout the peritoneal dialysate generation flow path if required. Water is pumped through the peritoneal dialysate generation cabinet **801** to a water purification module, shown as sorbent cartridge **812** in FIG.'s 7A-B. The water can enter the sorbent cartridge **812** through tubing (not shown) connected to the bottom of the sorbent cartridge **812** within the peritoneal dialysate generation cabinet **801**. The water exits the sorbent cartridge **812** through connector **813** and tubing **814**. An osmotic agent from osmotic agent source **815** and an ion concentrate from an ion concentrate source **817** are added to the fluid as described to generate non-sterilized peritoneal dialysate. The osmotic agent concentrate is added to the fluid through paddle connector **816**. The ion concentrate is added to the fluid through paddle connector **818**. A concentrate pump (not shown) can provide a driving force to move fluid from the concentrate sources into the peritoneal dialysate generation flow path inside of the peritoneal dialysate generation cabinet **801**. As described, the system can use a single ion concentrate source in place of the two sources shown in FIG.'s 7A-B, or more than two concentrate sources. The generated peritoneal dialysate can then be pumped through a sterilization module (not shown), such as an ultrafilter. A second ultrafilter and/or a UV light source can also be included. An integrated cycler (not shown in FIG.'s 7A-D) can then pump the dialysate into infusion line **819** through connector **820** and into the patient. Fitting **825** allows the infusion line **819** to be removed from the system for cleaning or replacement. Waste fluids can be pumped out of the system through waste line **807**, which connects to the peritoneal dialysate generation cabinet **801** through connector **830** having fitting **831**. A separate waste line for removing used dialysate from the patient (not shown in FIG.'s 7A-D) can also connect to the peritoneal dialysate generation cabinet **801** and connect to waste line **807**. The waste line **807** enters waste container **808** through a connector **829** in the top **809** of the waste container **808**. Handles **810** and **811** can be included on water source **804** and waste container **808** for easy movement and storage. Although the peritoneal dialysate generation cabinet **801** is illustrated on top of table **826** in FIG.'s 7A-D, the peritoneal dialysate generation cabinet **801** can be used on any stable flat surface.

As described, the peritoneal dialysate generation flow path can include various sensors for detection of conductivity, pH, refractive index, or other dialysate parameters. The sensors can be included either inside or outside of the body of the peritoneal dialysate generation cabinet **801**. The fluid lines and valves connecting the components of the peritoneal dialysate generation flow path can likewise be positioned inside of the cabinet body. As described, a top of the peritoneal dialysate generation cabinet **801** can have a graphical user interface **802** including screen **803**. Messages from the control system to the user, or from the user to the control system, can be generated and read through the graphical user interface **802**. The user can direct the generation of peritoneal dialysate through the graphical user interface **802**, and can receive messages from the system through screen **803**. The system can generate alerts to the user, including any problems detected by any of the sensors, as well as the progress of peritoneal dialysate generation. A handle **824** can be included for opening the peritoneal dialysate generation cabinet **801** to allow access to components on the inside of the cabinet. Handles **821** and **823** can be included to hold the fluid lines and power cord when not in use.

Disinfection connector **822** illustrated in FIG.'s 7A and 7C can be included for disinfection of the waste line **807**. During disinfection, the waste line **807** can be disconnected from waste container **808** and connected to disinfection connector **822**. Disinfectant solution from a disinfectant source (not shown in FIG.'s 7A-D) can then be circulated through the waste line **807** to disinfect the waste line **807**. Disinfection connector **827** can be included for disinfection of fluid line **805**. Fluid line **805** can be connected to disinfection connector **822** and disinfection solution can be circulated through the fluid line **805**. Drain **834** on water source **804** and drain **835** on waste container **808**, allow the water source **804** and waste container **808** to be drained without inverting the containers.

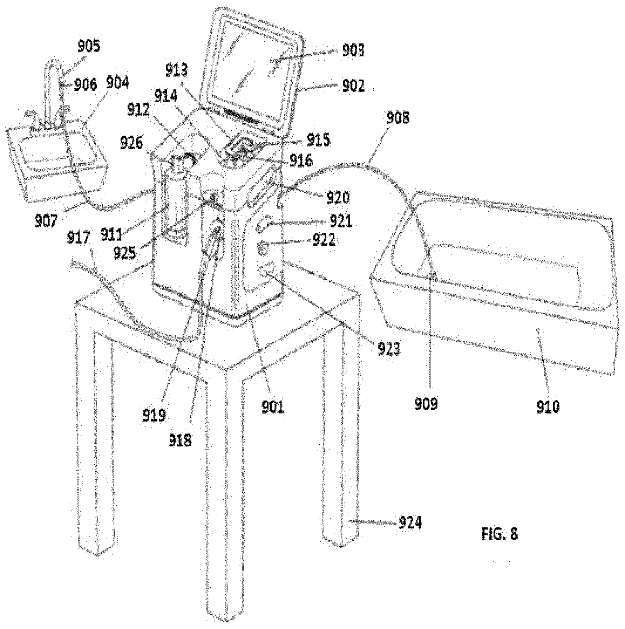

FIG. 8 illustrates a peritoneal dialysate generation cabinet **901** using a non-purified water source, faucet **905** in sink **904**. Although illustrated as faucet **905** and sink **904**, one of ordinary skill in the art will understand that any water source can be used. The ability to use municipal or other non-purified sources of water allow the peritoneal dialysate generation system to work at a patient's home without the need to store large amounts of purified water or dialysate. Fitting **906** connects the water line **907** to the faucet **905** or other water source, allowing the water line **907** to be connected or disconnected as necessary. A pump (not shown) can provide a driving force for the movement of fluid throughout the peritoneal dialysate generation flow path as described with respect to FIG.'s 1 and 5-6. The water is pumped through the peritoneal dialysate generation cabinet **901** to a water purification module, shown as sorbent cartridge **911** in FIG. 8. The water enters the sorbent cartridge **911** through tubing (not shown) connected to the bottom of the sorbent cartridge **911** within the peritoneal dialysate generation cabinet **901**. The water exits the sorbent cartridge **911** through connector **926** and tubing **912**. An osmotic agent from osmotic agent source **913** and an ion concentrate from an ion concentrate source **914** are added to the fluid as described to generate non-sterilized peritoneal dialysate. The osmotic agent concentrate is added to the fluid through paddle connector **916**. The ion concentrate is added to the fluid through paddle connector **915**. A concentrate pump (not shown) can provide a driving force to move fluid from the concentrate sources into the peritoneal dialysate generation flow path inside of the peritoneal dialysate generation cabinet **901**. As described, the system can use a single ion concentrate source in place of the two sources shown in FIG. 8, or more than two concentrate sources. The generated peritoneal dialysate can then be pumped through a sterilization module (not shown), such as an ultrafilter. A second ultrafilter and/or a UV light source can also be included. An integrated cycler (not shown in FIG. 8) can then pump the dialysate into infusion line **917** through connector **918** and into the patient. Fitting **919** allows the infusion line **917** to be removed from the system for cleaning or replacement. Waste fluids can be pumped out of the system through waste line **908**, which can connect to a drain **909** shown in bathtub **910**. A separate drain line (not shown) from the patient can be included to move used dialysate into the drain **909**. Although shown as a bathtub drain **909** in FIG. 8, the waste fluids can be conveyed to any type of drain, or alternatively to a waste container as illustrated in FIG.'s 7A-D. Although the peritoneal dialysate generation cabinet **901** is illustrated on top of table **924** in FIG. 8, the peritoneal dialysate generation cabinet **901** can be used on any stable flat surface. In certain embodiments, the peritoneal dialysate generation cabinet **901** and the patient can be in the same room as the water source and drain **909**. Alternatively, the patient and/or peritoneal dialysate generation cabinet **901** can be in a separate room, with tubing long enough to reach patient. For longer distances, the tubing should be strong enough to withstand the pressures necessary in pumping fluid over longer distances.

As described, a top of the peritoneal dialysate generation cabinet **901** can have a graphical user interface **902** including screen **903**. Messages from the control system to the user, or from the user to the control system, can be generated and read through the graphical user interface **902**. The user can direct the generation of peritoneal dialysate through the graphical user interface **902**, and can receive messages from the system through screen **903**. The system can generate alerts to the user, including any problems detected by any of the sensors, as well as the progress of peritoneal dialysate generation. A handle **920** can be included for opening the peritoneal dialysate generation cabinet **901** to allow access to components on the inside of the cabinet. Handles **921** and **923** can be included to hold the fluid lines and power cord when not in use.

Disinfection connector **922** can be included for disinfection of the waste line **908**. During disinfection, the waste line **908** can be disconnected from the drain **909** and connected to disinfection connector **922**. Disinfectant solution from a disinfectant source (not shown in FIG. 8) can then be circulated through the waste line **908** to disinfect the waste line **908**. Disinfection connector **925** can be included for disinfection of water line **907**. The water line **907** can be disconnected from faucet **905** and connected to disinfection connector **925**. Disinfectant solution can be circulated through the water line **907** for disinfection.

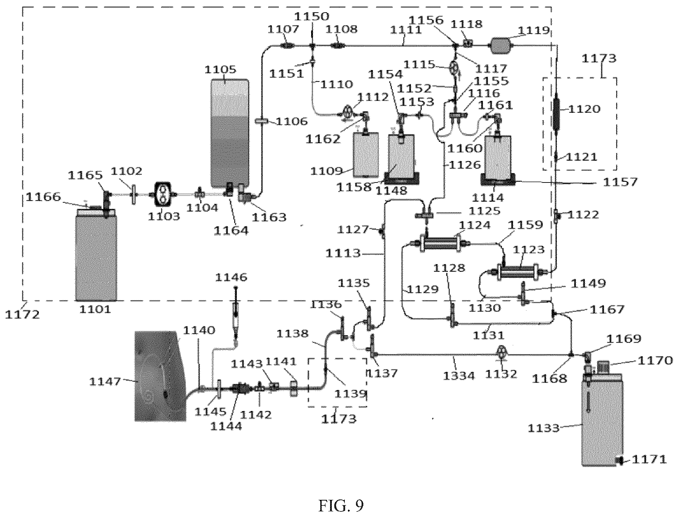

FIG. 9 illustrates an alternative non-limiting embodiment of a peritoneal dialysate generation flow path **1111**. Water from water source **1101** can be pumped into the peritoneal dialysate generation flow path **1111** by system pump **1103** through connector **1165**. Although shown with screw top **1166** in FIG. 9, any method can be used with the water source **1101** to fill and drain the water source **1101**. The water can be pumped through filter **1102** to remove any particulate matter from the water prior to entering the peritoneal dialysate generation flow path **1111**. Alternatively, a dedicated water source, such as a tap or a municipal water source, can be used in place of water source **1101**. Pressure sensor **1104** measures the pressure upstream of sorbent cartridge **1105**. In certain embodiments, an alternative water purification module can be used in place of sorbent cartridge **1105**, including a reverse osmosis module, a nanofilter, a combination of ion and anion exchange materials, activated carbon, silica, or silica based columns. The shading in sorbent cartridge **1105** shows varying layers of sorbent material. However, any order of sorbent material layers can be used, or the sorbent materials can be intermixed. In FIG. 9, the sorbent cartridge **1105** has a fluid inlet **1164** and fluid outlet **1163** in a base of the sorbent cartridge **1105**. In certain embodiments, the fluid inlet **1164** and fluid outlet **1163** can instead be on opposite sides of the sorbent cartridge **1105**. A filter **1106** can remove particulate matter in the fluid exiting sorbent cartridge **1105**.

A first conductivity sensor **1107** can measure the conductivity of the fluid exiting sorbent cartridge **1105**. One or more infusates can be added from infusate reservoir **1109** through connector **1162** to infusate line **1110** by infusate pump **1112** to the peritoneal dialysate generation flow path **1111** at T-junction **1150**. Filter **1151** can remove any particulate matter from the infusate concentrate prior to reaching the peritoneal dialysate generation flow path **1111**. Alternatively, a valve can be used in place of T-junction **1150**. A second conductivity sensor **1108** can measure the conductivity of the fluid after addition of the infusates to ensure proper concentrations of each infusate. As described, the system can include any number of infusate sources, each with the same or separate infusate pumps and infusate lines.

An osmotic agent pump **1115** can add an osmotic agent to the peritoneal dialysate generation flow path **1111** through osmotic agent line **1117** at T-junction **1156**. A filter **1152** can remove any particulate matter from the osmotic agent concentrate. As illustrated in FIG. 9, the system can have multiple osmotic agent sources, including dextrose source **1148** fluidly connected to osmotic agent line through connector **1154** and icodextrin source **1114** fluidly connected to osmotic agent line through connector **1160**. Filter **1153** can remove particulate matter from fluid exiting dextrose source **1148** and filter **1161** can remove particulate matter form fluid exiting icodextrin source **1114**. Alternative osmotic agent sources, including an amino acid source or a glucose source, can be used in place of, or in addition to, the dextrose source **1148** and icodextrin source **1114**, allowing customization of the osmotic agents used. Valve **1116** can control the source from which the osmotic agent is obtained. Alternatively, multiple osmotic agent lines and osmotic agent pumps can be started or stopped to prevent and direct flow to a desired flow path or component. A flow sensor **1118** measures the flow rate of fluid through the peritoneal dialysate generation flow path **1111**. A composition sensor **1119** can measure the concentrations of the osmotic agents in the fluid, as well as the infusates. The composition sensor can include a single sensor, or multiple sensors measuring separate fluid parameters.

Heater **1120** heats the fluid in the peritoneal dialysate generation flow path **1111** to the patient body temperature. Temperature sensor **1121** measures the temperature of the fluid and can be used to by a control system to control the heater **1120**, heating the fluid to a temperature of between around 25 °C to around 40°C. In a preferred embodiment, the desired temperature can be 37 ± 2 °C. A control system can monitor the temperature and shut off flow or generate an alarm if the temperature is outside of the desired range. In certain embodiments, the control system can shut off flow if the temperature is equal to greater than around 41 °C. Pressure sensor **1122** measures the pressure of the fluid prior to entering a dialysate sterilization module.

The dialysate sterilization module can include a first ultrafilter **1123** and a second ultrafilter **1124** fluidly connected by fluid line **1159**. The fluid flows through both ultrafilters to remove any chemical or biological contaminants. Waste fluid can exit the first ultrafilter **1123** through fluid line **1130** and exit the second ultrafilter **1124** through fluid line **1129**. Valves **1149** and **1128** control the movement of fluid between the first ultrafilter **1123** and second ultrafilter **1124** into waste line **1131**, which is fluidly connected to fluid line **1130** at T-junction **1167**. Valves **1149** and **1128** can be used to modulate the fluid movement out of ultrafilters **1123** and **1124** to ensure sufficient pressure for ultrafiltration. If the pressure in ultrafilter **1124** decreases below a necessary value, valve **1128** can be closed, preventing fluid movement from ultrafilter **1123** into fluid line **1130** and increasing the pressure in ultrafilter **1124**. The waste line **1131** is fluidly connected to a waste line **1134** at T-junction **1168** and to waste reservoir **1133** through connector **1169,** or alternatively, to a drain. Although shown with a screw top **1170** and tap **1171**, one of skill in the art will understand that alternative methods for filling and draining waste reservoir **1133** can be used.

Fluid exiting the second ultrafilter **1124** passes through valve **1125**. Valve **1125** can direct the fluid into either fluid line **1113** and an integrated cycler or into fluid line **1126** for addition to the dextrose source **1148** and icodextrin source **1114** via T-junction **1155**. The fluid can be added to dextrose source **1148** and icodextrin source **1114** to dissolve solid icodextrin and solid dextrose prior to generating the peritoneal dialysate.

Fluid line **1113** can include a pressure sensor **1127** to ensure that the fluid pressure is within predetermined limits prior to entering the integrated cycler. Valve **1135** controls the movement of fluid from the sterilization module. Valve **1136** controls the movement of fluid into and out of the integrated cycler through cycler line **1138**.

The cycler line **1138** can include a second temperature sensor **1139** to ensure the proper temperature of the peritoneal dialysate prior to infusion into the patient **1147**. An air detector **1141** is included to detect any air that would otherwise be introduced into the patient **1147**. A bubble trap (not shown) can be included to remove any detected air. A flow sensor **1143** measures the flow rate of fluid in the cycler line **1138** and can be used to control the amount of peritoneal dialysate infused into the patient **1147**. A pressure sensor **1142** can be included to ensure the fluid pressure in cycler line **1138** is within predetermined limits for infusion into the patient **1147**. A catheter **1140** can connect to the cycler line **1138** at connection **1144**. In certain embodiments, a heparin syringe **1146** can be included to add heparin or other medication to the peritoneal dialysate. Filter **1145** removes any particulate matter prior to infusion into the patient **1147**.

After a dwell period, the spent peritoneal dialysate can be drained from the patient **1147** through the cycler line **1138.** Drain pump **1132** can provide the driving force for draining the spent peritoneal dialysate. The spent peritoneal dialysate passes through valves **1136** and **1137** and into drain line **1134**, which can fluidly connect to waste reservoir **1133** or to a drain.

As illustrated in FIG. 9, the dialysate generation system can be fluidly connected to a temperature regulation system. The temperature regulation system includes the heater **1120**, and temperature sensors **1121** and **1139**, as well as a control system controlling the heater **1120**, shown as dashed boxes **1173**. The dialysate generation system includes the water source **1101**, infusate reservoir **1109**, and the one or more osmotic agent sources fluidly connected to the peritoneal dialysate generation flow path **1111**, shown as dashed box **1172**. The dialysate generation system can also include the sterilization module, including ultrafilters **1123** and **1124**, and the fluid lines connected to the integrated cycler. For patient comfort, the peritoneal dialysate can be heated to the patient body temperature prior to infusing the peritoneal dialysate into the patient. Heater **1120** heats the peritoneal dialysate to within a predetermined range of temperatures. In certain embodiments, the predetermined range can be between around 35 °C and around39°C. The dialysate temperature can initially be measured by temperature sensor **1121** downstream of heater **1120**. Prior to infusing the peritoneal dialysate, the temperature can be measured by temperature sensor **1139**, located in cycler line **1138**. The temperature sensors can be in communication with a control system (not shown). The control system can control the heater **1120** based on the temperatures measured by each of temperature sensor **1139** and **1121**. If the temperature is within the predetermined range as measured by temperature sensor **1121** and/or temperature sensor **1139**, the control system can pump the peritoneal dialysate into the peritoneal cavity of the patient **1147**. If the temperature system is outside of the predetermined range, the control system can stop infusing the peritoneal dialysate into the patient **1147**. The control system can also generate an alarm indicating the temperature of the peritoneal dialysate is outside of the predetermined range.

As illustrated in FIG. 9, the heater **1120** and first temperature sensor **1121** are positioned in a fluid line upstream of the purity control system including the first ultrafilter **1123** and second ultrafilter **1124**. In certain embodiments, a solid source of the osmotic agents and/or infusates can be used. The solid source can be placed in dextrose source **1148** and icodextrin source **1114**. To generate an osmotic agent concentrate, water from water source **1101** can be added to the dextrose source **1148** and icodextrin source **1114**, generating a concentrate of known concentration. To ensure that the icodextrin source **1114** and dextrose source **1148** remain free from chemical or biological contamination, the water can first be passed through the purity control system, including first ultrafilter **1123** and second ultrafilter **1124** prior to addition to the osmotic agent sources. Water from water source **1101** can be pumped through sorbent cartridge **1105** and peritoneal dialysate generation flow path **1111**. Heater **1120** heats the water and is controlled by a control system based on data received from temperature sensor **1121**. The control system can control heater **1120** to heat the water to a set temperature, which will affect the solubility of the osmotic agents and allow the osmotic agent concentrate to be of known concentration. The water is then pumped through first ultrafilter **1123**, through fluid line **1159** and second ultrafilter **1124**. Valve **1125** can direct the water from the second ultrafilter **1124** through fluid line **1126** and into each of icodextrin source **1114** and dextrose source **1148** via osmotic agent line **1117** to generate osmotic agent concentrates free from contamination. As described, the peritoneal dialysate generation system can include any number of osmotic agent sources wherein the second ultrafilter **1124** can be fluidly connected to each osmotic agent source. Although not illustrated in FIG. 9, the second ultrafilter **1124** can also be fluidly connected to the infusate reservoir **1109** to generate an infusate concentrate free from chemical or biological contamination. Heater **1120** can heat the water prior to passing through the first ultrafilter **1123** and second ultrafilter **1124**. Using heated water to dissolve the osmotic agents can allow for a faster and more complete dissolution in order to minimize system preparation time before treatment can begin. In certain embodiments, the water can be heated to between around 25°C to around90°C to minimize dissolution time of the PD fluid components, while also minimizing formation of glucose degradation products. The osmotic agent concentrate can then be mixed with the purified water in the peritoneal dialysate generation flow path **1111** and the temperature can be diluted down by the incoming stream. Temperature sensor **1121** can be used to measure the temperature of the water to ensure that the water is within the predetermined range. The control system can control the heater **1120** based on the temperature measured by temperature sensor **1121** to maintain the water within the predetermined range while dissolving the osmotic agents. Optional Vibration plate **1157** can agitate the solution in icodextrin source **1114**, and optional vibration plate **1158** can agitate the solution in dextrose source **1148** to further speed dissolution of the osmotic agents. One of skill in the art will understand that alternative means of agitation can be used, including stirrers or other mixers.

After use, the system can be cleaned and disinfected. A disinfectant source (not shown), such as citric acid or bleach, can be fluidly connected to the peritoneal dialysate generation system. In certain embodiments, the sorbent cartridge **1105** can be removed and a disinfectant source attached in place of the sorbent cartridge **1105**. In other embodiments, the disinfectant source can be fluidly connected at any location in the peritoneal dialysate generation system. In certain embodiments, a closed loop can be made by connecting the peritoneal dialysate generation flow path **1111** at a location downstream of the water source **1101** to the waste line **1134**. A connector to the water source **1101** can be fluidly connected to a connector for the waste reservoir **1133** to form a closed loop. The system pump **1103** can recirculate the disinfectant solution from the disinfectant source through all of the fluid lines and components of the peritoneal dialysate generation system. During disinfection, the disinfectant solution can be heated and the temperature regulated with the temperature regulation system to improve the efficiency of disinfection. Heater **1120** can heat the disinfectant solution to a desired temperature. Temperature sensors **1121** and **1139** can measure the temperature of the recirculating disinfectant solution. A control system can control the heater based on the temperature measured by temperature sensors **1121** and **1139** to regulate the temperature and maintain the temperature within a predetermined range. In certain embodiments, the predetermined range can be between around 50°C and 99°C. If the temperature drops below the predetermined range, the control system can activate heater **1120** to further heat the solution. If the temperature rises above the predetermined range, the control system can deactivate heater **1120** to allow the temperature of the disinfectant solution to drop.

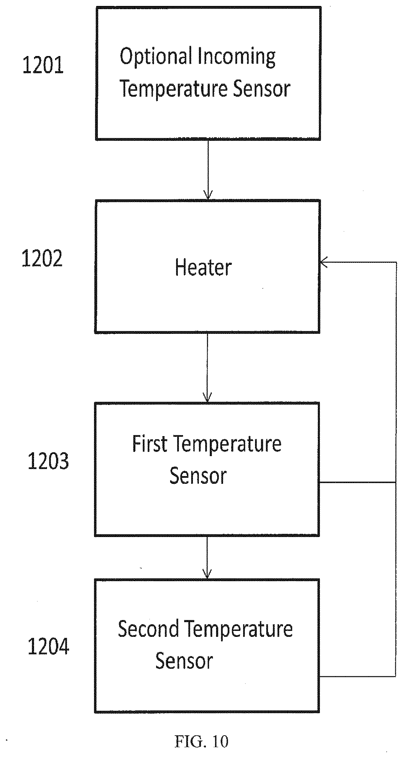

FIG. 10 illustrates a non-limiting embodiment of the control of the temperature regulation system. In certain embodiments, an optional incoming temperature sensor **1201** can be included to measure the temperature of the fluid prior to reaching the heater **1202.** The incoming temperature sensor **1201** can provide additional control information and can be used to determine how much to module the heater **1202,** providing better overall control. A higher temperature of incoming fluid would require less heating by the heater **1202.** The heater **1202** heats the fluid to a desired temperature. A first temperature sensor **1203** downstream of the heater **1202** can measure the temperature of the fluid. A control system (not shown) can receive the temperature from temperature sensor **1203** and control the heater **1202** to maintain the temperature in a desired temperature range. A second temperature sensor **1204** can measure the temperature of the fluid prior to infusion into the patient. In certain embodiments, temperature sensor **1203** can serve as the primary feedback control for the heater **1202** as temperature sensor **1203** is closer to the heater **1202** in the flow path. The second temperature sensor **1204** can serve as a final check of the temperature prior to infusion into the patient, providing a "fine-tuning" adjustment to the temperature accounting for any temperature drop in the flow path downstream of temperature sensor **1203.** Second temperature sensor **1204** also serves as a final safety check for the fluid prior to infusion into the patient. Second temperature sensor **1204** provides feedback in very close proximity to the point of fluid delivery to the patient and thus allows for fine tuning and very precise temperature control because second temperature sensor **1204** allows for compensation for temperature loss/gain in the fluid path between first temperature sensor **1203** and second temperature sensor **1204.** The feedback times for temperature control based on the second temperature sensor **1204** may be longer than that of temperature sensor **1203** due to the longer flow loop volume between the heater and temperature sensor **1204.** The expected temperatures as measured by temperature sensors **1203** and **1204** can be between 34-40°C, but temperature sensors **1203** and **1204** can be capable of measuring a much wider temperature range and would provide feedback to the safety system. For example, if the temperature is outside of a predetermined range, flow can be stopped or an alarm generated.

One skilled in the art will understand that various combinations and/or modifications and variations can be made in the described systems and methods depending upon the specific needs for operation. Moreover, features illustrated or described as being part of an aspect of the invention may be used in the aspect of the invention, either alone or in combination, or follow a preferred arrangement of one or more of the described elements.

The invention may be described by reference to the following numbered paragraphs:-
1. A temperature regulation system for use in peritoneal dialysis, comprising:
   a first fluid line fluidly connected to a dialysate generation system; the first fluid line including a heater and a first temperature sensor positioned downstream of the heater;
   the first fluid line fluidly connectable to an integrated cycler through a second fluid line having a second temperature sensor;
   a control system in communication with the first temperature sensor, the second temperature sensor, and the heater; the control system controlling the heater based on a temperature measured by the first temperature sensor and the second temperature sensor.
2. The temperature regulation system of paragraph 1; wherein the first fluid line is fluidly connected to at least one osmotic agent source downstream of the heater.
3. The temperature regulation system of paragraph 2; further comprising a sterilization module positioned between the first fluid line and the osmotic agent source.
4. The temperature regulation system of paragraph 2; wherein the sterilization module comprises one or more ultrafilters.
5. The temperature regulation system of paragraph 1, the dialysate generation system fluidly connectable to a disinfectant source.
6. The temperature regulation system of paragraph 5, the dialysate generation system fluidly connectable to a waste line; the waste line fluidly connectable to the integrated cycler.
7. The temperature regulation system of paragraph 6, further comprising a system pump; the system pump recirculating fluid from the disinfectant source, through the dialysate generation system, the temperature regulation system, and the second fluid line.
8. The temperature regulation system of paragraph 7, the control system controlling the heater based on a temperature measured by the first temperature sensor and/or the second temperature sensor.
9. A method, comprising the steps of:
   a) generating a peritoneal dialysate with a peritoneal dialysate generation system;
   b) pumping the peritoneal dialysate through a temperature regulation system; the temperature regulation system comprising at least a heater and a first temperature sensor;
   c) pumping the peritoneal dialysate into an integrated cycler through at least one fluid line; the at least one fluid line having a second temperature sensor;
   d) measuring a temperature of the peritoneal dialysate in the at least one fluid line; and
   e) heating the peritoneal dialysate with the heater to a desired temperature as measured by the first temperature sensor and/or the second temperature sensor.
10. The method of paragraph 9, wherein the desired temperature as measured by the first temperature sensor is between around 25 °C and around 40 °C.
11. The method of paragraph 9, wherein the desired temperature as measured by the second temperature sensor is between around 25 °C and around 40 °C.
12. The method of paragraph 9, further comprising the step of pumping the peritoneal dialysate from the integrated cycler.
13. The method of paragraph 12, further comprising the step of generating an alarm if a temperature of the peritoneal dialysate as measured by the first temperature sensor and/or the second temperature sensor is outside of a predetermined range.
14. The method of paragraph 12, further comprising stopping the step of pumping the peritoneal dialysate from the integrated cycler if a temperature of the peritoneal dialysate as measured by the first temperature sensor and/or the second temperature sensor is outside of a predetermined range.
15. The method of paragraph 13, wherein the predetermined range as measured by the first temperature sensor is between around 25 °C and around 40 °C.
16. The method of paragraph 13, wherein the predetermined range as measured by the second temperature sensor is between around 25 °C and around 40 °C.
17. A method of disinfecting a peritoneal dialysis system, comprising the steps of:
   a) fluidly connecting a disinfectant source to a first fluid line of a peritoneal dialysate generation system; wherein the peritoneal dialysate generation system is fluidly connected to a temperature regulation system and a second fluid line; the second fluid line fluidly connectable to an integrated cycler;
   b) fluidly connecting the first fluid line to the second fluid line;
   c) recirculating a disinfectant solution through the peritoneal dialysis system;
   d) measuring a temperature of the disinfectant solution in the peritoneal dialysis system; and
   e) heating the disinfectant solution to a desired temperature as measured by a first temperature sensor positioned in the first fluid line downstream of a heater and/or a second temperature sensor positioned in the second fluid line.
18. The method of paragraph 17, wherein the desired temperature as measured by the first temperature sensor is equal to or greater than around 80 °C.
19. The method of paragraph 17, wherein the desired temperature as measured by the second temperature sensor is equal to or greater than around 80 °C.
20. The method of paragraph 17, wherein the disinfectant solution is a citric acid solution.

## Claims

1. A temperature regulation system for use in peritoneal dialysis, comprising:
a first fluid line fluidly connected to a dialysate generation system; the first fluid line including a heater and a first temperature sensor positioned downstream of the heater;
the first fluid line fluidly connectable to an integrated cycler through a second fluid line having a second temperature sensor;
a control system in communication with the first temperature sensor, the second temperature sensor, and the heater; the control system controlling the heater based on a temperature measured by the first temperature sensor and the second temperature sensor.

2. The temperature regulation system of claim 1; wherein the first fluid line is fluidly connected to at least one osmotic agent source downstream of the heater.

3. The temperature regulation system of claim 2; further comprising a sterilization module positioned between the first fluid line and the osmotic agent source, preferably wherein the sterilization module comprises one or more ultrafilters.

4. The temperature regulation system of any preceding claim, the dialysate generation system fluidly connectable to a disinfectant source.

5. The temperature regulation system of claim 4, the dialysate generation system fluidly connectable to a waste line; the waste line fluidly connectable to the integrated cycler.

6. The temperature regulation system of claim 5, further comprising a system pump; the system pump recirculating fluid from the disinfectant source, through the dialysate generation system, the temperature regulation system, and the second fluid line.

7. The temperature regulation system of any preceding claim, wherein the control system controlling the heater based on a temperature measured by the first temperature sensor and/or the second temperature sensor.

8. A method, comprising the steps of:
a) generating a peritoneal dialysate with a peritoneal dialysate generation system;
b) pumping the peritoneal dialysate through a temperature regulation system; the temperature regulation system comprising at least a heater and a first temperature sensor;
c) pumping the peritoneal dialysate into an integrated cycler through at least one fluid line; the at least one fluid line having a second temperature sensor;
d) measuring a temperature of the peritoneal dialysate in the at least one fluid line; and
e) heating the peritoneal dialysate with the heater to a desired temperature as measured by the first temperature sensor and/or the second temperature sensor.

9. The method of claim 8, wherein the desired temperature as measured by the first temperature sensor is between around 25 °C and around 40 °C , or wherein the desired temperature as measured by the second temperature sensor is between around 25 °C and around 40 °C, or wherein the desired temperature as measured by both the first temperature sensor and the second temperature sensor is between around 25 °C and around 40 °C.

10. The method of any of claims 8 to 9, further comprising the step of pumping the peritoneal dialysate from the integrated cycler.

11. The method of claim 10, further comprising the step of generating an alarm if a temperature of the peritoneal dialysate as measured by the first temperature sensor and/or the second temperature sensor is outside of a predetermined range, preferably wherein the predetermined range as measured by the first temperature sensor is between around 25 °C and around 40 °C and wherein the predetermined range as measured by the second temperature sensor is between around 25 °C and around 40 °C.

12. The method of claim 10 or 11, further comprising stopping the step of pumping the peritoneal dialysate from the integrated cycler if a temperature of the peritoneal dialysate as measured by the first temperature sensor and/or the second temperature sensor is outside of a predetermined range, preferably wherein the predetermined range as measured by the first temperature sensor is between around 25 °C and around 40 °C and wherein the predetermined range as measured by the second temperature sensor is between around 25 °C and around 40 °C.

13. A method of disinfecting a peritoneal dialysis system, comprising the steps of:
a) fluidly connecting a disinfectant source to a first fluid line of a peritoneal dialysate generation system; wherein the peritoneal dialysate generation system is fluidly connected to a temperature regulation system and a second fluid line; the second fluid line fluidly connectable to an integrated cycler;
b) fluidly connecting the first fluid line to the second fluid line;
c) recirculating a disinfectant solution through the peritoneal dialysis system;
d) measuring a temperature of the disinfectant solution in the peritoneal dialysis system; and
e) heating the disinfectant solution to a desired temperature as measured by a first temperature sensor positioned in the first fluid line downstream of a heater and/or a second temperature sensor positioned in the second fluid line.

14. The method of claim 13, wherein the desired temperature as measured by the first temperature sensor is equal to or greater than around 80 °C.

15. The method of claim 13 or claim 14, wherein the desired temperature as measured by the second temperature sensor is equal to or greater than around 80 °C.
